# FASCICULE DE BREVET EUROPEEN

(11) **EP 0 668 737 B1**
(45) Date de publication et mention de la délivrance du brevet: **19.03.1997**
(21) Numéro de dépôt: 94900185.3
(22) Date de dépôt: 10.11.1993
(51) Int. Cl.: A47K 11/02, B64D 11/02, A61B 19/02

(54) **DISPOSITIF DE RECUPERATION ET DE STOCKAGE DE DECHETS**
VORRICHTUNG ZUM SAMMELN UND LAGERN VON ABFÄLLEN
WASTER RECOVERY AND STORAGE DEVICE

(30) Priorité: 13.11.1992 FR 9213917
(43) Date de publication de la demande: 30.08.1995
(73) Titulaire: INNOVATION - INGENIERIE - INTEGRATION - SYSTEME, F-31450 Montgiscard (FR)
(72) Inventeur: HENGL, Patrick, F-31450 Montgiscard (FR)
(74) Mandataire: Cabinet BARRE LAFORGUE & associés
(86) Numéro de dépôt international: FR9301107
(87) Numéro de publication internationale: WO9410893

(56) Documents cités:
- WO-A-88/02614
- DE-A- 2 457 093
- GB-A- 1 294 129
- US-A- 3 452 368
- US-A- 3 643 266
- US-A- 3 648 302
- US-A- 3 723 999

## Description

L'invention concerne un dispositif de récupération et de stockage de déchets. Elle s'applique notamment à la récupération de déchets à risques tels que les déchets hospitaliers. Une autre application consiste en la réalisation d'un dispositif de récupération et de stockage destiné à servir de cabinet d'aisances.

La récupération et le stockage de déchets, notamment de déchets à risques, tels que déchets hospitaliers, se fait le plus souvent de façon empirique en utilisant des poubelles qui, faute d'être vidées et nettoyées régulièrement, constituent la source de dangers non négligeables.

Par ailleurs, et notamment dans le domaine aéronautique, la récupération et le stockage des déchets que constituent les excréments humains posent de gros problèmes notamment de stockage donnant lieu, à l'heure actuelle, à de nombreuses recherches visant par exemple à solutionner ces problèmes au moyen d'un séchage par microondes...

La présente invention vise à pallier ces inconvénients et a pour principal objectif de fournir un dispositif solutionnant l'ensemble des problèmes susévoqués inhérents à la récupération et au stockage de déchets.

Un autre objectif de l'invention est de fournir un dispositif conçu pour pouvoir fonctionner de façon autonome.

Un autre objectif de l'invention est de fournir un dispositif permettant d'assurer le traitement des déchets, notamment dans l'application cabinet d'aisances.

A cet effet, l'invention vise un dispositif récupérateur de déchets comprenant :
- un réceptacle doté d'une ouverture d'introduction des déchets,
- une gaine s'étendant à l'intérieur du réceptacle au travers de l'ouverture,
- un obstacle de changement de direction disposé en aval de l'ouverture d'introduction transversalement par rapport à la gaine, ledit obstacle étant agencé pour se trouver sur le trajet de ladite gaine de façon à incurver la trajectoire de celle-ci,
- la gaine étant disposée autour du réceptacle à l'extérieur de ce dernier, et s'invagine à l'intérieur dudit réceptacle au travers de l'ouverture, ladite gaine présentant une section inférieure à celle du réceptacle et étant apte à s'expanser radialement,

Selon l'invention, ce dispositif se caractérise en ce que :
- des moyens d'entraînement de la gaine sont adaptés pour coopérer avec un tronçon de celle-ci se trouvant en aval de l'obstacle de changement de direction et pour :
   . maintenir constamment en tension la portion de gaine située en amont desdits moyens d'entraînement de façon à provoquer une fermeture de ladite gaine au contact de l'obstacle de changement de direction,
   . entraîner, à la commande, un déplacement de l'ensemble de la gaine sur une distance prédéterminée, de façon à amener un nouveau tronçon de gaine à pénétrer dans le réceptacle,
- des moyens d'actionnement sont associés aux moyens d'entraînement,
- et une zone de stockage de la gaine est disposée en aval des moyens d'entraînement.

Grâce à sa conception, un tel dispositif constitue un "siphon mécanique" qui permet "d'avaler" et d'isoler immédiatement les déchets déposés dans le réceptacle.

En effet, la gaine constamment maintenue en tension du fait de son élasticité radiale et de la conception des moyens d'entraînement, se referme au droit de l'obstacle de changement de direction qui constitue la barrière au-delà de laquelle le déchet est isolé.

En outre, la portion de gaine située au niveau de l'ouverture du réceptacle et pouvant donc se trouver en contact avec une source de contamination, est renouvelée à chaque opération d'évacuation.

Il est à noter que le brevet US-3 643 266 décrit un dispositif récupérateur de déchets, du type défini dans le préambule de la revendication 1 de la présente demande. Toutefois, un tel dispositif "statique" ne saurait permettre de remplir les objectifs, notamment du point de vue hygiènique, visés par l'invention.

En effet, du fait de la conception de ce dispositif, la partie de la poche disposée dans le réceptacle reste toujours souillée par les déchets introduits dans ce dernier. De plus, lorsque le réceptacle est relevé manuellement en vue d'évacuer les déchets vers le fond de la poche, l'exécutant est soumis aux odeurs des déchets se trouvant dans ce fond de poche.

Selon une autre caractéristique de l'invention, le réceptacle présente une section tubulaire comportant une section croissante s'évasant en direction de l'ouverture d'introduction.

Cette forme conique de l'entrée du réceptacle permet de garantir un dépliage radial total de la gaine et ce, quelle que soit la façon dont cette gaine a été conditionnée autour du réceptacle.

Par ailleurs, selon un mode de réalisation préférentiel, ce dispositif comprend avantageusement :
- un dévidoir de section tubulaire disposé entre l'obstacle de changement de direction et les moyens d'entraînement, et agencé pour que la gaine passe à l'intérieur dudit dévidoir,
- une deuxième gaine étanche stockée autour du dévidoir à l'extérieur de ce dernier et adaptée pour se dévider autour de la première gaine, de façon à ensacher ladite première gaine et à être entraînée avec celle-ci par les moyens d'entraînement.

Cet ensachage de la première gaine à l'intérieur d'une gaine étanche représente une barrière supplémentaire bactériologique.

En outre, selon une autre caractéristique de l'invention, le dispositif comprend une pompe apte à être commandée par les moyens d'actionnement et dotée d'une entrée d'aspiration reliée à un réservoir destiné à renfermer un produit antiseptique, et une sortie de refoulement reliée à un injecteur disposé dans le réceptacle.

La vaporisation d'un produit antiseptique constitue une désinfection active qui assure une première barrière bactériologique et offre notamment la possibilité d'assimiler les déchets hospitaliers ensachés selon l'invention à des déchets ménagers.

Selon un premier mode de réalisation préférentiel, les moyens d'entraînement comprennent avantageusement :
- des moyens de traction adaptés pour entraîner un déplacement de la gaine sur une distance prédéterminée, de façon à amener un nouveau tronçon de gaine à pénétrer dans le réceptacle,
- des moyens de stockage situés dans la zone de stockage et comportant une bobine d'envidage portée par un arbre de rotation, et des moyens d'entraînement en rotation de ladite bobine aptes à la faire tourner d'une distance angulaire équivalant à la distance linéaire de déplacement de la gaine, lors de chaque actionnement des moyens de traction.

Selon ce mode de réalisation, le dispositif comporte des moyens de stockage qui, d une part, permettent de maintenir la gaine en tension, et d'autre part, présentent l'avantage d'être d'une manipulation aisée et sécurisante lors de leur chargement et lors de leur transfert en vue, par exemple, d'un traitement ultérieur.

Dans ce cas, en outre, les moyens de traction comprennent de façon avantageuse :
- deux rouleaux transversaux disposés de façon à se trouver de part et d'autre de la gaine, lesdits rouleaux étant solidaires, au niveau de chacune de leurs extrémités, d'un axe excentré par rapport à leur axe de symétrie longitudinal,
- des moyens d'entraînement des rouleaux, commandés par les moyens d'actionnement et aptes à déplacer ensemble lesdits rouleaux le long de la gaine entre une position amont et une position aval,
- des moyens de rappel aptes à ramener les rouleaux de leur position aval vers leur position amont,
- des moyens de pivotement des axes excentrés des rouleaux aptes à maintenir lesdits rouleaux :
   . en contact l'un contre l'autre lors de leur déplacement de leur position amont vers leur position aval, de façon à entraîner la gaine pincée entre lesdits rouleaux,
   . éloignés l'un de l'autre lors de leur retour de leur position aval vers leur position amont.

De plus, selon une autre caractéristique de l'invention, les moyens de pivotement comprennent, pour chaque rouleau, un levier solidaire de l'axe excentré dudit rouleau, et des organes de butée amont et aval aptes à entraîner la rotation dudit levier. De plus, des moyens élastiques bistables sont associés aux leviers et agencés pour maintenir les rouleaux dans l'une ou l'autre de leur position relative, après pivotement desdits leviers.

En outre, les moyens d'entraînement en rotation de la bobine comprennent avantageusement un moteur ressort précontraint dans une phase préalable et adapté pour provoquer une rotation de ladite bobine lorsque les rouleaux sont dans leur position éloignés l'un de l'autre.

Un dispositif ainsi conçu présente l'avantage de pouvoir être entièrement autonome en utilisant par exemple, en tant que moyens d'actionnement, un organe, tel qu'une pédale reliée par une tringlerie aux rouleaux.

Selon un deuxième mode de réalisation préférentiel, les moyens d'entraînement comprennent avantageusement :
- deux rouleaux transversaux portés chacun par un arbre de rotation, disposés de part et d'autre de la gaine de façon que celle-ci soit maintenue pincée entre lesdits rouleaux,
- des moyens d'entraînement en rotation des rouleaux, commandés par les moyens d'actionnement, aptes à chaque commande, à faire tourner lesdits rouleaux de façon unidirectionnelle et en sens inverse, d'une distance angulaire prédéterminée,
- des moyens de basculement de l'arbre de rotation d'au moins un des rouleaux, aptes à permettre aux rouleaux de s'écarter l'un de l'autre,
- et des moyens de rappel reliant les arbres de rotation des rouleaux, de façon à exercer une force tendant à maintenir ces derniers en contact l'un contre l'autre.

Selon ce mode de réalisation, la traction de la gaine et son maintien en tension sont réalisés au moyen d'un seul ensemble comportant comme éléments de base deux rouleaux agencés pour maintenir la gaine pincée.

De tels moyens d'entraînement présentent l'avantage de former l'équivalent d'un laminoir qui écrase et compacte les déchets, le passage desdits déchets étant rendu possible grâce à la faculté d'écartement relatif que présentent les rouleaux.

En outre, en vue d'améliorer le compactage sans nuire à l'entraînement de la gaine, les rouleaux comportent avantageusement une face périphérique présentant des cannelures s'étendant selon les génératrices desdits rouleaux.

Selon un mode de réalisation préférentiel concernant ces moyens d'entraînement :
- l'arbre de rotation d'un des rouleaux, dit fixe, est monté fixe en translation, l'arbre de l'autre rouleau, dit mobile, étant solidaire de leviers aptes à permettre un basculement dudit rouleau mobile par rapport au rouleau fixe,
- les moyens d'entraînement en rotation des rouleaux comprennent des moyens d'entraînement unidirectionnel du rouleau fixe, associés aux moyens d'actionnement, et des organes de transmission aptes à transmettre ce mouvement de rotation, de façon inversée, au rouleau mobile.

De tels organes de transmission comprennent, en outre, avantageusement :
- une poulie à deux gorges montée de façon coaxiale par rapport à l'axe de rotation des leviers,
- deux poulies solidaires chacune de l'arbre de rotation d'un des rouleaux,
- deux courroies reliant chacune la poulie d'un des rouleaux à la poulie à deux gorges, et disposées de façon à inverser le sens de rotation du rouleau mobile par rapport à celui du rouleau fixe.

Par ailleurs, deux modes de réalisation préférentiels sont envisagés selon l'invention, concernant l'obstacle de changement de direction.

Selon une première variante de réalisation cet obstacle de changement de direction est constitué d'un coude disposé dans le prolongement de l'ouverture et agencé pour que la gaine se referme au contact de la portion supérieure dudit coude.

Ce premier mode de réalisation est particulièrement adapté mais non spécifiquement réservé, à la fabrication d'un dispositif dans lequel les différents éléments sont disposés les uns à la suite des autres.

Dans ce cas, ces éléments, à savoir réceptacle, moyens de traction, moyens de stockage sont préférentiellement logés à l'intérieur d'un carter, le réceptacle et les moyens de stockage étant de plus montés de façon amovible dans ledit carter.

Afin de posséder ce caractère amovible, le réceptacle comporte, avantageusement, deux axes transversaux, fixés extérieurement sur et sous ledit réceptacle vers son extrémité opposée à l'ouverture d'introduction. Parallèlement, le carter possède quatre glissières agencées pour recevoir à coulissement les axes du réceptacle, et dotées, vers leur extrémité, d'organes de blocage en translation desdits axes.

En outre, le dévidoir est préférentiellement adapté pour venir se loger sur sa plus grande longueur dans le réceptacle, en aval du coude, et comporte deux axes transversaux fixés extérieurement sur et sous ledit dévidoir, vers une des extrémités de ce dernier, lesdits axes, de longueur inférieure aux axes du réceptacle étant portés au niveau de chacune de leurs extrémités par une pièce amovible de liaison avec un axe du réceptacle. Il est à noter que la gaine étanche est alors stockée autour du dévidoir de façon à s invaginer à l'intérieur de ce dernier.

De plus, en vue de l'amovibilité de la bobine, le carter comporte avantageusement deux gorges inclinées par rapport à la verticale aptes à loger les extrémités de l'arbre de rotation de la bobine.

Selon une autre caractéristique de l'invention, le dispositif comprend un chariot apte à porter le carter, doté d'un habillage comportant un capot amovible percé d'une ouverture conjuguée de l'ouverture d'introduction du réceptacle.

De plus, dans le cas où l'on désire une très grande asepsie, ce dispositif peut également intégrer, de façon avantageuse, des moyens d'aspiration aptes à créer une dépression à l'intérieur de l'habillage, et des moyens de filtration de l'air refoulé par lesdits moyens d'aspiration.

Selon la deuxième variante de réalisation concernant l'obstacle de changement de direction, ce dernier est constitué de deux axes parallèles dits amont et aval, disposés transversalement à distance l'un de l'autre de façon que lors du passage de la gaine entre lesdits axes, la trajectoire de cette dernière soit incurvée de telle manière que ladite gaine se referme au contact de l'axe aval.

Ce deuxième mode de réalisation est particulièrement adapté mais non spécifiquement réservé à la fabrication d'un dispositif dans lequel le réceptacle, les moyens d'entraînement de la gaine et la zone de stockage sont agencés les uns en dessous des autres.

Un tel dispositif comporte, en outre, avantageusement, une coque dotée d'une ouverture supérieure, apte à loger le réceptacle et les moyens d'entraînement, et à délimiter dans sa partie inférieure une zone de stockage accessible, le réceptacle étant monté de façon amovible dans ladite coque.

De plus, en vue de permettre un changement aisé des deux gaines et de façon préférentielle :
- la gaine étanche est stockée autour du dévidoir de façon à se dévider dans le prolongement inférieur dudit dévidoir, autour de la première gaine sortant de ce dernier,
- le dévidoir présente une collerette supérieure agencée pour permettre de suspendre ledit dévidoir à l'intérieur du carter doté à cet effet d'éléments supports de ladite collerette,
- le réceptacle est agencé pour venir reposer sur la collerette du dévidoir.

Selon une autre caractéristique de l'invention, le réceptacle présente une section tubulaire ovoïde et comporte une portion supérieure de section croissante s'évasant en direction de l'ouverture d'introduction, et une portion inférieure de section constante. De plus, les axes s'étendent selon la plus grande dimension du réceptacle, l'axe amont étant disposé dans la portion supérieure dudit réceptacle, et l'axe aval étant disposé dans la portion inférieure et étant relié à l'axe amont de façon à pouvoir basculer relativement à ce dernier.

Ce basculement de l'axe aval a pour avantage d'augmenter la taille des déchets pouvant être absorbés.

Le dispositif selon l'invention est également conçu pour pouvoir servir de cabinet d'aisances. Dans ce cas, de façon avantageuse :
- le réceptacle est conformé, en aval du coude, de façon à constituer un bac de récupération de liquides, doté d'une sortie d'évacuation desdits liquides,
- la première gaine présente une texture adaptée pour laisser s'écouler les liquides au travers de ladite gaine vers le bac de récupération,
- des moyens de traitement sont adaptés pour assurer le traitement des liquides récupérés en vue de permettre leur rejet et/ou leur utilisation.

Un tel dispositif permet, en premier lieu, de solutionner les problèmes d'encombrement que posent les cabinets d'aisances actuels dans le domaine aéronautique. De plus, il assure une séparation des excréments qui permet de stocker les seules matières solides, (fèces, papier...) et de traiter les liquides de façon à autoriser, en fin de traitement, un rejet de ces derniers ou leur utilisation, par exemple, pour se laver les mains. Il est à noter, à cet effet, que la récupération des impuretés se trouvant dans les liquides se fait de façon prépondérante dans le bac du réceptacle qui est amovible et donc très facilement nettoyable.

De plus, l'ouverture du réceptacle est recouverte d'un tronçon de gaine qui est automatiquement renouvelé entre chaque utilisation.

Selon un mode de réalisation préférentiel, les moyens de traitement comprennent des moyens de centrifugation des liquides, des moyens de pressurisation des liquides récupérés après centrifugation, et des moyens de filtration vers lesquels les liquides sont délivrés sous pression.

De plus, ce dispositif est préférentiellement logé dans une enceinte comportant des moyens d'aspiration aptes à créer une dépression à l'intérieur de ladite enceinte, et des moyens de filtration de l'air refoulé par lesdits moyens d'aspiration.

Outre leur encombrement réduit, cette enceinte et le matériel renfermés présentent un poids notablement réduit par rapport aux installations existantes, ce qui correspond à un gros avantage par rapport à ces dernières dans un domaine, l'aéronautique, où tout gain de poids est fortement apprécié.

D'autres caractéristiques, buts et avantages de l'invention ressortiront de la description détaillée qui suit en référence aux dessins annexés qui en représentent à titre d'exemples non limitatifs trois modes de réalisation préférentiels. Sur ces dessins qui font partie intégrante de la présente description :
- la figure 1 est une vue en perspective d'un chariot équipé d'une première variante de dispositif conforme à l'invention,
- la figure 2 est une vue en perspective, avec des arrachés partiels, de cette première variante de dispositif conforme à l'invention,
- la figure 3 est une coupe longitudinale par un plan vertical A,
- la figure 4 est une vue en coupe, à échelle agrandie, des moyens de traction de ce dispositif, illustrant les deux positions amont (en traits pointillés) et aval (en traits pleins) de ces moyens de traction,
- la figure 5 est une coupe transversale par un plan vertical B de cette première variante de dispositif conforme à l'invention,
- les figures 6a à 6d sont des schémas illustrant le fonctionnement de ce dispositif,
- la figure 7 est une vue en perspective d'un module vertical équipé d'une deuxième variante de dispositif conforme à l'invention,
- la figure 8 est une vue frontale partiellement en coupe par un plan vertical de ce module,
- la figure 9 est une coupe longitudinale par un plan transversal C de ce module,
- la figure 10 est une coupe longitudinale partielle par un plan transversal D de ce module,
- les figures 11 à 11c sont des schémas illustrant le fonctionnement de ce module,
- et la figure 12 est un schéma fonctionnel d'un dispositif conforme à l'invention utilisé en tant que cabinet d'aisances.

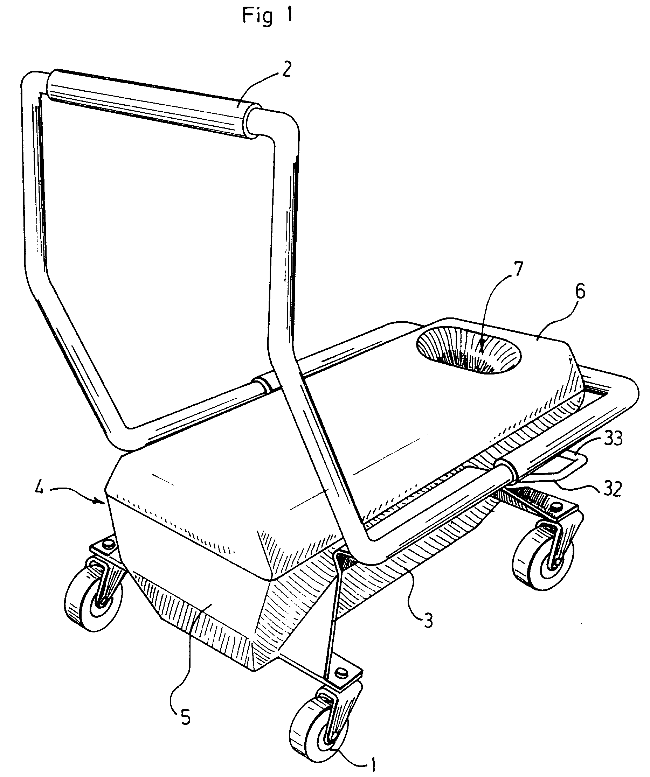

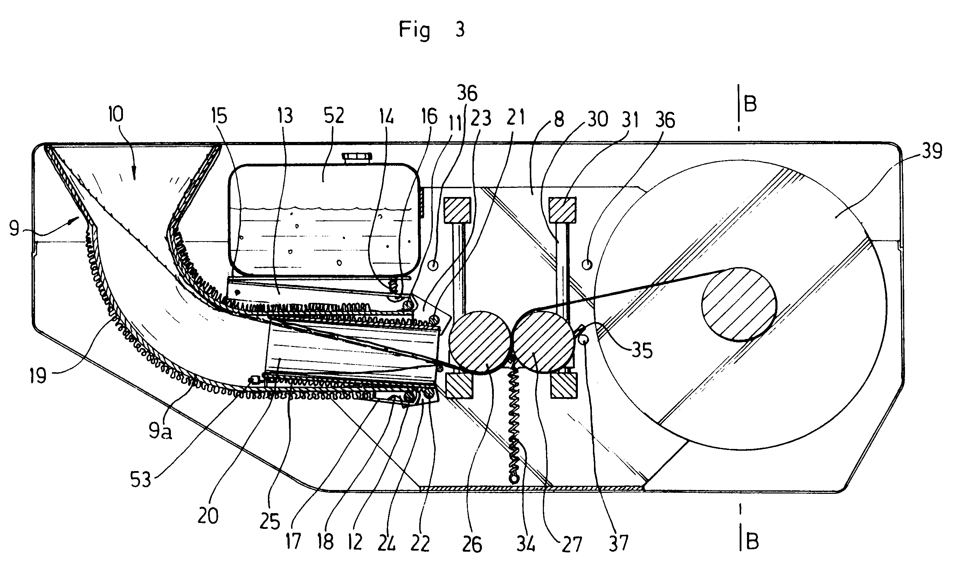

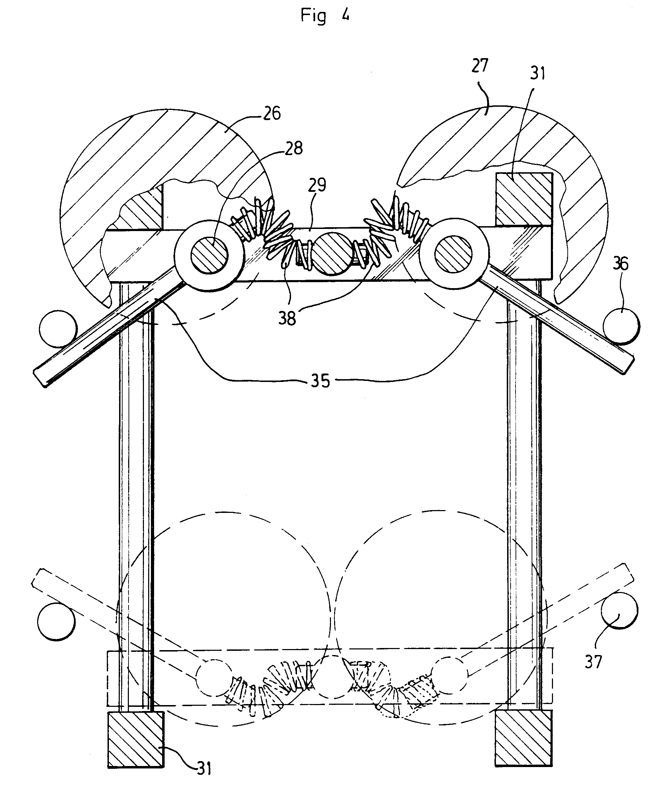

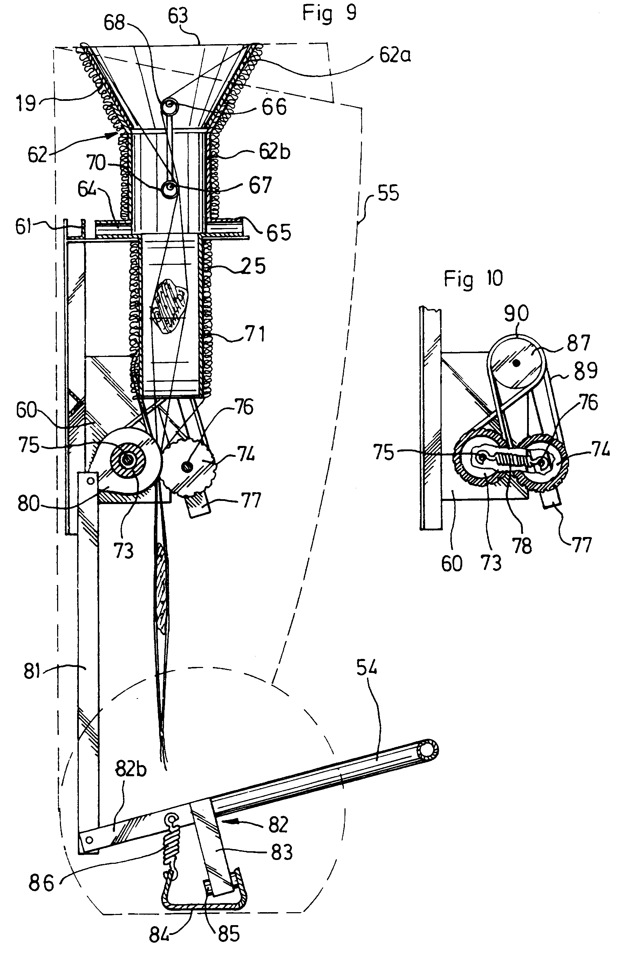

Les dispositifs représentés aux figures 1 à 5 et 7 à 10 consistent respectivement en un chariot et un module vertical destinés à la récupération, au traitement et au stockage de déchets, notamment dans le milieu médical. Ces dispositifs sont conçus pour fonctionner de façon autonome sans nécessiter l'apport d'une quelconque énergie.

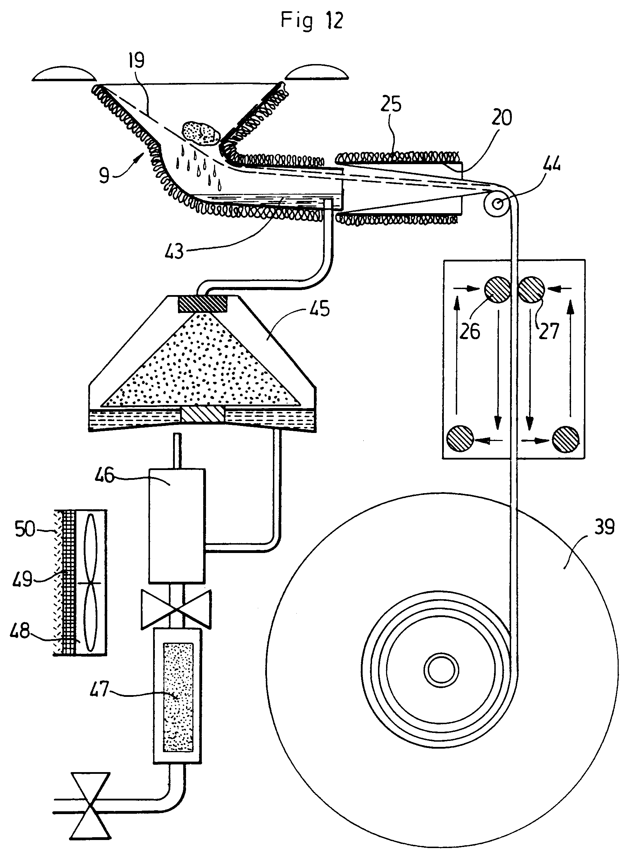

Le dispositif représenté à la figure 12 constitue, quant à lui, un cabinet d'aisances particulièrement destiné à être utilisé dans le domaine aéronautique.

Le dispositif représenté à la figure 1 consiste en un chariot monté sur roulettes 1 et doté d'une barre de manoeuvre 2. Ce chariot comporte un châssis 3 portant un habillage 4 composé d'une coque 5 fixée sur le châssis 3 et d'un capot amovible 6. Ce capot 6 est, en outre, percé d'une ouverture 7 de forme ovoïde constituant l'ouverture d'introduction des déchets.

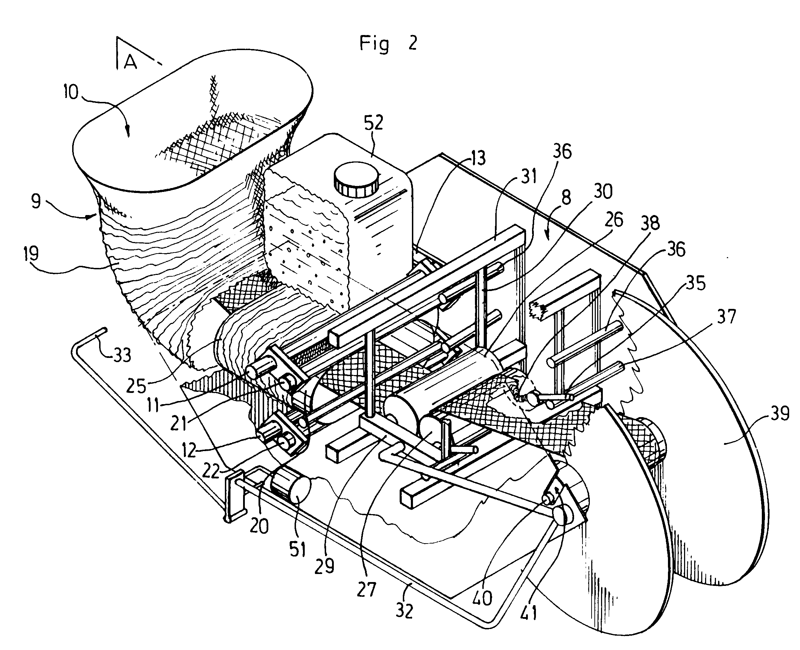

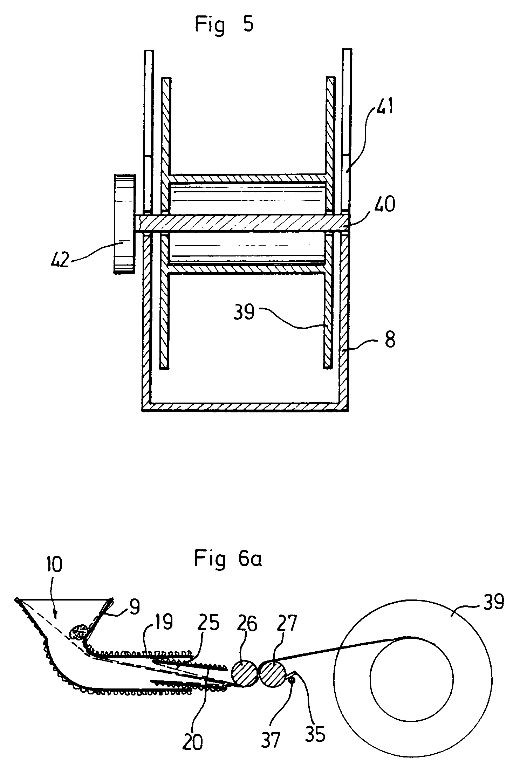

Le dispositif de récupération des déchets est, quant à lui, intégré à l'intérieur de l'habillage et, tel que représenté aux figures 2 et 5, logé à l'intérieur d'un carter 8 constitué d'une tôle présentant transversalement une forme générale de U.

Ce dispositif comprend, en premier lieu, un réceptacle amovible 9 constitué d'un élément tubulaire en acier inoxydable de section ovoïde à grand axe horizontal, présentant longitudinalement une forme coudée.

Ce réceptacle 9 est doté d'une ouverture 10 d'introduction des déchets et se trouve disposé de façon que cette ouverture 10 soit en regard de l'ouverture 7 du capot 6. Il présente, en outre, entre le coude 9a et cette ouverture 10, une section croissante lui conférant une forme d'entonnoir.

Le réceptacle 9 comporte, en outre, deux axes transversaux 11, 12 fixés extérieurement sur et sous ledit réceptacle, au niveau de son extrémité opposée à l'ouverture 10.

En vue du maintien de ce réceptacle, le dispositif comporte quatre glissières en forme de L fixées deux à deux en regard sur les faces internes de chacune des ailes du carter 8, et destinées à loger, chacune, une extrémité d'un axe transversal 11, 12.

Ces glissières comportent, en premier lieu, deux glissières supérieures 13 articulées sur les ailes du carter 8, au niveau de leur extrémité d'introduction des axes 11. Chacune de ces glissières supérieures 13 est, en outre, maintenue inclinée par rapport à l'horizontale en pente descendante selon le sens d'introduction des axes 11, au moyen d'un ressort 14, interposé entre l'extrémité de ladite glissière opposée à l'axe d'articulation et une plate-forme horizontale 15 disposée au-dessus de cette dernière.

Chaque glissière 13 comporte, enfin, un organe 16 de blocage en translation de l'axe 11, disposé de façon à faire saillie à l'intérieur de ladite glissière, à proximité de son extrémité opposée à l'axe d'articulation.

Les glissières comportent, en second lieu, deux glissières inférieures fixes 17 également disposées selon une pente descendante, et destinées à loger les axes inférieurs 12 du réceptacle 9. De même que les glissières supérieures 13, ces glissières inférieures 17 comportent un organe de blocage 18 des axes 12.

Le dispositif comporte, en outre, une gaine 19 en un matériau textile extensible radialement, resserrée radialement en position normale. Cette gaine qui constitue un élément consommable présente une longueur de plusieurs mètres, et est destinée à être disposée en accordéon autour du réceptacle 9, et à s invaginer à l'intérieur de ce dernier au travers de l'ouverture 10.

Il est à noter que, grâce à la forme "en entonnoir" du réceptacle, la gaine 19 est parfaitement tendue et lisse au droit de l'ouverture 10.

Le dispositif comporte par ailleurs, un dévidoir 20 constitué d'un élément tubulaire rectiligne de section ovoïde de dimensions inférieures à celles du réceptacle 9, adapté pour venir se loger sur sa plus grande longueur à l'intérieur de ce réceptacle 9.

Ce dévidoir 20 comporte au niveau d'une de ses extrémités, deux axes transversaux 21, 22 de longueur inférieure à celle des axes 11, 12, fixés extérieurement sur et sous ledit dévidoir.

Il est relié au réceptacle 9 au moyen de deux pièces de liaison supérieure 23 et inférieure 24 percées chacune de deux orifices agencés pour loger respectivement un des axes 11, 12 du réceptacle 9, et un des axes 21, 22 du dévidoir 20.

Le dispositif comporte, en outre, une deuxième gaine 25 en un matériau plastique constituant un deuxième élément consommable. Cette gaine 25 est destinée à être disposée en accordéon autour du dévidoir 20 et à s'invaginer à l'intérieur de ce dernier de façon à ensacher la gaine textile 19.

Le dispositif comporte également des moyens de traction adaptés pour engendrer, sur commande, un déplacement simultané d'une dizaine de centimètres des deux gaines 19, 25.

Ces moyens de traction comprennent, en premier lieu, deux rouleaux transversaux 26, 27 disposés horizontalement et entre lesquels les gaines 19-25 sont amenées à passer. Ces deux rouleaux 26, 27 sont dotés, à chacune de leurs extrémités, d'un axe 28 excentré par rapport à leur axe de symétrie longitudinal.

Ces rouleaux 26, 27 sont, en outre, montés entre deux barres horizontales, telles que 29, dotées de trous borgnes agencés pour loger leurs axes 28 tout en permettant la rotation desdits axes. Ces deux barres 29 sont, quant à elles, montées coulissantes le long de colonnes verticales telles que 30, portées entre deux barres transversales horizontales telles que 31 s'étendant entre les deux ailes du carter 8.

Les moyens de traction comprennent, en outre, des moyens d'entraînement en translation des rouleaux 26, 27 aptes à engendrer un coulissement vers le haut des barres 29 le long des colonnes 30.

Ces moyens d'entraînement consistent en une tringlerie 32 constituée de deux éléments disposés de part et d'autre du carter 8 et articulés sur les ailes de ce dernier, lesdits éléments étant reliés vers une de leurs extrémités par une tringle transversale 33 formant une pédale accessible à l'avant du chariot, et étant solidarisés vers leur autre extrémité à une des barres 29.

Les moyens de traction comprennent, de plus, des moyens de rappel aptes à engendrer un coulissement vers le bas des barres 29 le long des colonnes 30, lorsque la pression sur la pédale 33 est relâchée. Ces moyens de rappel consistent en des ressorts 34 agencés verticalement et solidarisés au carter 8 et à une barre 29.

Les moyens de traction comprennent enfin des moyens de pivotement des rouleaux 26, 27 aptes à maintenir ces derniers :
- en contact l'un contre l'autre lors de leur déplacement vers le haut, engendré par une action sur la pédale 33, de façon qu'ils entraînent les gaines 19, 25 pincées entre eux,
- éloignés l'un de l'autre lors de leur retour vers leur position basse engendrée par les ressorts 34.

Ces moyens de pivotement comprennent, en premier lieu, des leviers 35 solidaires des axes 28 de chaque rouleau 26, 27, et s'étendant orthogonalement par rapport à ces derniers.

Les moyens de pivotement comprennent, en outre, pour chaque rouleau 26, 27, deux tronçons de barres transversales 36, 37 fixées orthogonalement sur les ailes du carter 8, de butée respectivement haute et basse, aptes à entraîner la rotation des leviers lorsque ces rouleaux 26, 27 parviennent à leurs positions haute et basse.

Les moyens de pivotement comprennent enfin des ressorts bistables 38 reliant les leviers 35 aux barres 29, et adaptés pour maintenir lesdits leviers dans des positions correspondant aux positions accolées ou éloignées des rouleaux 26, 27 après rotation de ces derniers.

Le dispositif comporte, en outre, en aval des rouleaux 26, 27, une bobine interchangeable 39 de stockage des gaines 19, 25. Cette bobine 39 est dotée d'un axe de rotation 40 dépassant de part et d'autre des flasques de ladite bobine et venant se loger dans des gorges 41 inclinées par rapport à un axe vertical ménagées dans chacune des ailes du carter.

Cette bobine 39 est, en outre, associée à un moteur ressort 42 solidaire de son axe de rotation 40. Ce moteur ressort 42 précontraint dans une phase préalable, est adapté pour entraîner une rotation de la bobine 39 lorsque les rouleaux 26, 27 se trouvent éloignés l'un de l'autre, et ainsi entraîner l'envidage de la longueur des gaines 19, 25 tractée par ces rouleaux.

Enfin, le tambour de la bobine 39 est revêtu d'un dispositif d'accrochage, tel qu'une bande "Velcro", permettant la fixation de l'extrémité des gaines 19, 25 après un remplacement de bobine.

En dernier lieu, le dispositif comprend des moyens de désinfection active comportant une pompe mécanique 51 agencée pour être actionnée par la tringlerie 32 lorsque la pédale 33 est manoeuvrée.

Cette pompe 51 est raccordée côté aspiration à un réservoir 52 renfermant un produit antiseptique, disposé sur la plate-forme 15, et côté refoulement à un injecteur 53 disposé dans le réceptacle 9, qui permet d'imprégner de produit antiseptique la portion de gaine 19 entourant le déchet introduit dans ledit réceptacle.

Le fonctionnement du dispositif décrit ci-dessus est illustré aux figures 6a à 6d.

En position de repos illustrée à la figure 6a, les rouleaux 26, 27 sont en position basse et sont en contact l'un contre l'autre maintenant les gaines 19, 25 pincées le long d'une ligne transversale.

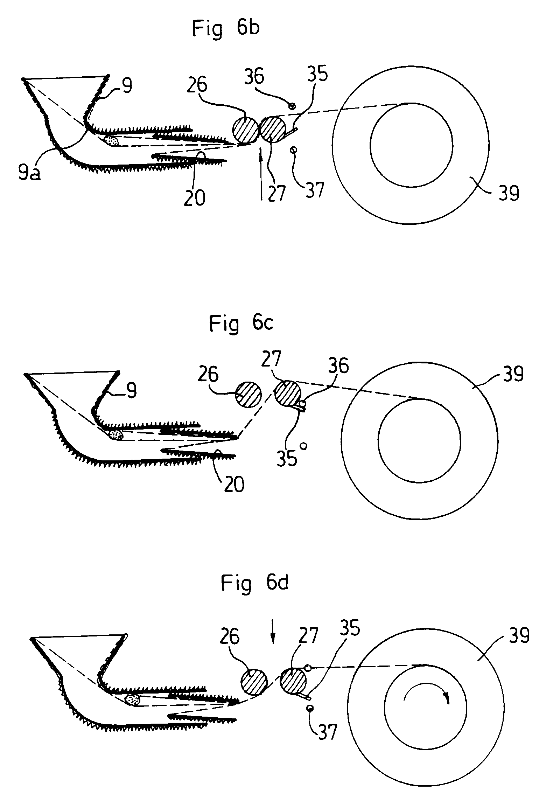

Après introduction d'un déchet, une action sur la pédale 33 entraîne un déplacement vers le haut des rouleaux 26, 27 accolés, et provoque ainsi un déplacement des gaines 19, 25 (figure 6b). Ce déplacement provoque "l'avalement" du déchet qui est amené en aval du coude 9a du réceptacle 9 et se trouve donc isolé du milieu extérieur.

En outre, lorsque les rouleaux 26, 27 parviennent en fin de course, les leviers 35 viennent en butée haute contre les barres transversales 36 et provoquent une rotation desdits rouleaux entraînant leur éloignement relatif (figure 6c).

Ainsi, lorsque la pédale 33 est relâchée et que les rouleaux 26, 27 redescendent sous l'action des ressorts 34, la bobine 39 peut absorber, sous l'action du moteur ressort 42, la longueur de gaines 19, 25 préalablement tractée (figure 6d).

Par ailleurs, le déchet ainsi "avalé" est imprégné de produit antiseptique soit immédiatement, soit lors d'une action ultérieure sur la pédale effectuée pour la récupération d'un autre déchet. De plus, lors d'actions ultérieures sur la pédale 33, la portion de gaine 19 entourant ce déchet est ensachée à l'intérieur de la gaine plastique 25.

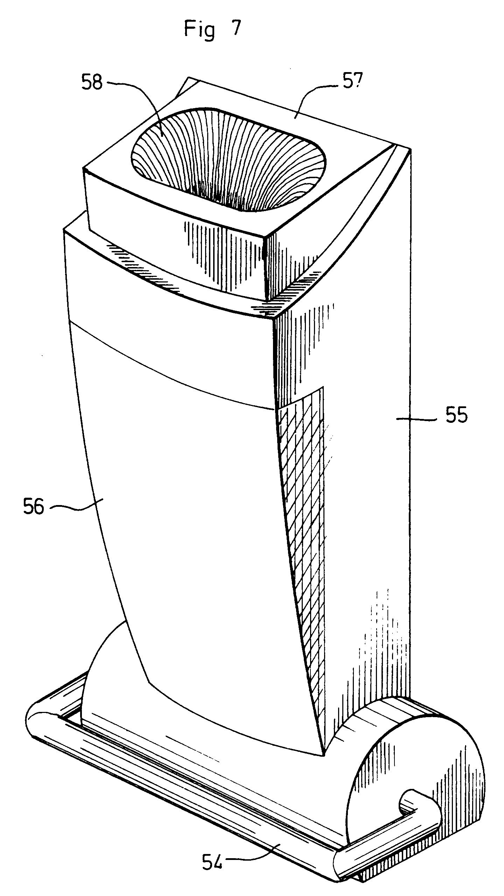

Le dispositif représenté à la figure 7 consiste en un module vertical doté d'une barre de manoeuvre 54 en partie basse. Ce module se compose d'une coque autoporteuse 55 dotée d'une paroi frontale 56 et d'un couvercle 57 d'un seul tenant montés pivotants autour d'un axe vertical. Le couvercle 57 est, en outre, percé d'une ouverture 58 de forme ovoïde constituant l'ouverture d'introduction des déchets.

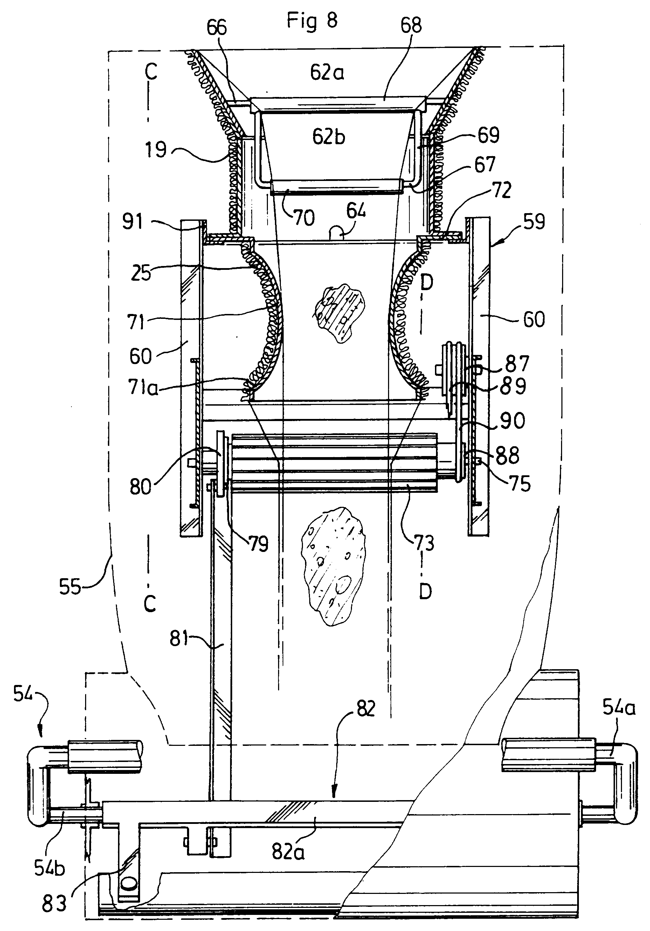

Le dispositif de récupération des déchets est, quant à lui, intégré à l'intérieur de la coque 55 et, tel que représenté aux figures 8 à 10, monté sur un châssis 59 comprenant principalement deux joues latérales telles que 60 reliées par des traverses frontales telles que 61.

Ce dispositif de récupération comprend, en premier lieu, un réceptacle amovible 62 constitué de deux éléments tubulaires de section ovoïde réalisés à base de résines, destinés à venir s'emboîter l'un dans l'autre. Ce réceptacle 62 comporte ainsi un tronçon supérieur 62a évasé vers le haut, se terminant par une ouverture 63 d'introduction des déchets de forme conjuguée de l'ouverture 58 de la coque 55.

Ce réceptacle 62 comporte également un tronçon inférieur 62b de forme ovoïde de section constante dans lequel vient s'emboîter le tronçon supérieur 62b. Il comporte, enfin, deux orifices tels que 64 ménagés en regard en partie inférieure respectivement des parois frontale et arrière du tronçon inférieur 62b, lesdits orifices étant prolongés extérieurement par deux manchons horizontaux tels que 65.

Comme précédemment, ce réceptacle 62 est habillé d'une gaine 19 disposée en accordéon autour de ce dernier, et destinée à s'invaginer à l'intérieur de celui-ci au travers de l'ouverture 63. Cette gaine 19 est initialement disposée autour du seul tronçon inférieur 62b qui constitue donc avec cette gaine un élément consommable, le tronçon supérieur 62a n'ayant pas, quant à lui, à être interchangé.

Par ailleurs, le réceptacle 62 intègre deux axes transversaux, dits amont 66 et aval 67 disposés horizontalement à l'intérieur dudit réceptacle, et s'étendant selon la plus grande dimension et dans un des plans de symétrie de ce dernier.

L'axe amont 66 s'étend dans la portion supérieure 62a du réceptacle 62 et est solidarisé, au niveau de chacune de ses extrémités, à la paroi dudit réceptacle, par exemple en noyant lesdites extrémités dans la résine.

Cet axe amont 66 est, en outre, logé sur une grande partie de sa longueur à l'intérieur d'un tube 68 libre en rotation et en translation par rapport audit axe.

L'axe aval 67 s'étend, quant à lui, dans la portion inférieure 62b du réceptacle 62, et forme l'âme d'un U dont les extrémités supérieures des ailes 69 sont soudées sur le tube 68. Ainsi agencé, cet axe aval 67 est suspendu au tube 68 et peut basculer de part et d'autre du plan de symétrie du réceptacle 62 et permettre ainsi le passage de déchets de taille supérieure à celle que permettrait un axe fixe.

De plus, cet axe aval 67 est également logé, sur sa plus grande longueur, à l'intérieur d'un tube 70 destiné à réduire les forces de frottement entre la gaine 19 et ledit axe.

Le dispositif comprend, en outre, un dévidoir 71 destiné à être disposé dans le prolongement inférieur du réceptacle 62, et à servir de support à ce dernier.

A cet effet, ce dévidoir 71 présente une face supérieure de dimensions sensiblement inférieures à celles du tronçon inférieur 62b du réceptacle, ladite face étant dotée d'une collerette externe 72 agencée pour porter ledit réceptacle.

Le dévidoir 71 est lui-même suspendu entre les deux joues 60 et repose par l'intermédiaire de sa collerette externe 72 sur l'aile horizontale de cornières 91 dont l'aile verticale est solidarisée auxdites joues.

Le dévidoir 71 présente, en outre, une section tubulaire rectangulaire, et comporte deux parois latérales 71a présentant longitudinalement une forme concave.

Comme précédemment, ce dévidoir 71 est habillé d'une gaine étanche 25 en un matériau plastique destinée à se dévider dans le prolongement inférieur dudit dévidoir, de façon à ensacher la première gaine 19. Il est à noter, à cet effet, que la forme concave des faces latérales 71a du dévidoir permet de stocker une plus grande longueur de gaine 25 autour dudit dévidoir.

Le dispositif comporte également des moyens d'entraînement aptes à engendrer à la commande un déplacement simultané d'une dizaine de centimètres des deux gaines 19, 25.

Ces moyens d'entraînement comprennent, en premier lieu, deux rouleaux transversaux 73, 74 accolés longitudinalement, montés chacun autour d'un arbre de rotation 75, 76, et disposés horizontalement de façon que les gaines 19, 25 soient amenées à passer entre eux.

Ces deux rouleaux 73, 74 destinés à maintenir les gaines par pincement présentent, en outre, des faces périphériques dotées de cannelures s'étendant selon leur génératrices, aptes à engréner les unes dans les autres.

Par ailleurs, l'arbre de rotation 75 d'un de ces rouleaux 73, dit rouleau fixe, s'étend entre les joues 60 auxquelles il est solidarise en translation.

Par contre, l'arbre de rotation 76 de l'autre rouleau 74, dit rouleau mobile, s'étend entre deux leviers 77 articulés sur les joues 60 de façon à pouvoir pivoter autour d'un axe horizontal et ainsi permettre au rouleau mobile 74 de basculer relativement au rouleau fixe 73.

De plus, les axes de rotation 75, 76 des deux rouleaux 73, 74 sont reliés par un ressort 78 tendant à s'opposer au basculement du rouleau mobile 74, et tendant par conséquent à maintenir lesdits rouleaux en contact l'un contre l'autre.

Les moyens d'entraînement des gaines 19, 25 comprennent également des moyens d'entraînement en rotation des rouleaux 73, 74 aptes à les faire tourner en sens inverse et de façon unidirectionnelle par actionnement de la barre de manoeuvre 54.

Ces moyens d'entraînement en rotation sont associés au rouleau fixe 73, des organes de transmission étant disposés entre ledit rouleau fixe et le rouleau mobile 74, en vue de transmettre en l'inversant, le mouvement de rotation.

Ces moyens d'entraînement en rotation comprennent, en premier lieu, un organe de transmission unidirectionnel tel qu'un système de roue libre, schématisé en 79, solidaire d'une des extrémités de l'arbre de rotation 75 du rouleau fixe 73.

Ils comprennent, en outre, une came de renvoi 80 agencée pour coopérer avec l'organe de transmission 79, ladite came de renvoi étant solidaire de l'extrémité supérieure d'une bielle verticale 81 adaptée pour entraîner la rotation de cette came lors de ses débattements longitudinaux.

Les moyens d'entraînement en rotation comprennent enfin un système d'actionnement reliant la barre de manoeuvre 54 à l'extrémité inférieure de la bielle 81. Il est a noter, à cet effet, que la barre de manoeuvre 54 présente la forme d'un U dont les ailes sont prolongées de retour orthogonaux auxdites ailes, l'âme dudit U formant une traverse d'actionnement 54a, disposée en avant de la coque 55, et les retours formant deux axes de pivotement 54b alignés s'étendant au travers d'orifices ménagés dans les parois latérales de ladite coque.

Le système d'actionnement comprend un levier 82 doté d'une traverse 82a s'étendant entre les retours 54b de la barre de manoeuvre 54 et solidarisés à ceux-ci, et d'une branche 82b orthogonale à la traverse 82a et articulée, vers son extrémité, sur la bielle 81.

Ce levier 82 comprend, en outre, un pied de butée 83, orthogonal à la traverse 82a et à la branche 82b, agencé pour limiter le débattement dudit levier, en association avec un sabot de butée 84.

A cet effet, le sabot de butée 84 présente une section ayant la forme d'un U dont les ailes sont sensiblement inclinées l'une vers l'autre, et l'extrémité du pied 83 du levier 82 est logé à l'intérieur dudit sabot, de façon à voir sa course limitée par les ailes de ce dernier. L'extrémité de ce pied 83 est, en outre, dotée de tampons en caoutchouc 85 disposés de façon à s'interposer entre ledit pied et les ailes du sabot 84.

Le système d'actionnement comprend, enfin, un ressort 86 disposé entre la branche 82b du levier et une des ailes du sabot 84, et adapté pour ramener la bielle vers sa position basse une fois la pression sur la barre de manoeuvre 54 relâchée.

Les organes de transmission disposés entre le rouleau fixe 73 et le rouleau mobile 74 comprennent, quant à eux, en premier lieu, une poulie à double gorge 87 solidaire d une joue 60 et coaxiale avec l'axe de pivotement d'un des leviers 77.

Ces organes de transmission comprennent, en outre, deux poulies telles que 88, solidaires chacune d'un rouleau 73, 74. Ils comprennent, enfin, deux courroies 89, 90 d'entraînement respectivement direct (montage normal) et inversé (montage en huit) reliant, chacune, la poulie à double gorge 87 à une des poulies 88.

Ce dispositif est également équipé d'une pompe et d'un réservoir (non représentés) similaires à ceux du dispositif constituant la première variante de réalisation, la pompe étant agencée pour être actionnée lorsque la barre de manoeuvre 54 est abaissée, et les injecteurs étant logés dans les manchons 65 du réceptacle.

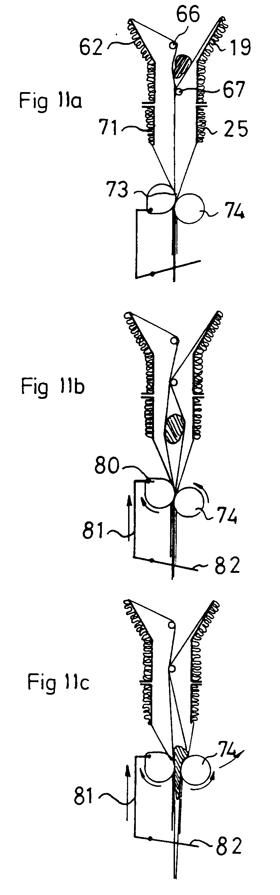

Le fonctionnement du dispositif décrit ci-dessus est illustré aux figures 11a à 11c.

En position de repos illustrée à la figure 11a, la gaine 19 est maintenue en tension entre l'ouverture 63 du réceptacle et les rouleaux 73, 74, et se referme au contact de l'axe aval 67.

Après introduction d'un déchet, une action sur la barre de manoeuvre 54 provoque un pivotement du levier 82 qui génère un déplacement vers le haut de la bielle 81, et par conséquent une rotation de la came de renvoi 80 transmise par l'organe de transmission unidirectionnel 79 au rouleau fixe 73 (figure 11b).

Ce mouvement de rotation est, en outre, transmis en sens inverse au rouleau mobile 74 par l'intermédiaire du système poulies 87, 88/courroies 89, 90.

Cette rotation des rouleaux 73, 74 provoque un déplacement linéaire des gaines 19, 25 maintenues pincées entre lesdits rouleaux, et par conséquent le déchet se trouve "avalé" en aval de l'axe aval 67, et isolé du milieu extérieur du fait que la gaine 19 constamment maintenue en tension se referme au droit dudit axe aval.

Une fois la pression sur la barre de manoeuvre 54 relâchée, le levier 82 est amené à pivoter sous l'action du ressort 86, générant un déplacement vers le bas de la bielle 81 et par conséquent une rotation de la came de renvoi 80. Par contre, l'organe de transmission 79 étant unidirectionnel, le rouleau 73 ne subit aucune rotation et la tension de la gaine 19 est maintenue.

Par ailleurs, tel qu'illustré à la figure 11c, lorsque le déchet parvient entre les deux rouleaux 73, 74, le rouleau mobile 74 est amené à basculer de façon à ménager un espace suffisant pour permettre le passage dudit déchet. Toutefois, ce basculement est limité par l'action du ressort 78 qui tend à rapprocher les rouleaux 73, 74 et qui provoque l'écrasement du déchet entre lesdits rouleaux qui font donc office de laminoirs.

Le dispositif représenté schématiquement à la figure 7 est de même conception que celui décrit en référence aux figures 1 à 5 mais vise une application différente, à savoir la réalisation d'un cabinet d'aisances notamment adapté à l'aéronautique.

Dans cette application, le réceptacle 9 fait office de cuvette du cabinet d'aisances et est conformé de façon à constituer un bac 43 de récupération des liquides s'écoulant au travers de la gaine 19.

Le dévidoir 20 est quant à lui disposé en aval du réceptacle 9, et un rouleau de renvoi 44 est disposé en aval de ce dévidoir 20 permettant de limiter l'encombrement en longueur du cabinet d'aisances.

Les moyens de traction et la bobine 39 sont quant à eux identiques à ceux décrits en référence aux figures 1 à 5, la seule différence résultant de l'inversion du sens de déplacement des rouleaux 26, 27.

Pour cette application, le dispositif comporte, en outre, une centrifugeuse 45 vers laquelle sont délivrés les liquides récupérés dans le bac 43 et déjà épurés par décantation, une chambre 46 de mise en pression des liquides épurés par centrifugation, et des moyens de filtration 47 vers lesquels les liquides sont injectés sous pression.

Comme procédé de filtration, on peut prévoir notamment une ultrafiltration, une filtration tangentielle ou une filtration osmotique.

Le dispositif comporte, par ailleurs, un ventilateur 48 destiné à mettre en dépression l'enceinte que constitue le cabinet d'aisances, et deux filtres, bactériologique 49 et à charbon actif 50 disposés au niveau du refoulement du ventilateur 48.

Un tel dispositif, de faibles poids et encombrement, permet de récupérer les excréments solides et autres matières solides (papier...) sur la bobine de stockage 39, et de rejeter directement les excréments liquides après traitement, ou de réutiliser ces derniers, notamment pour se laver les mains.

## Revendications

1. Dispositif récupérateur de déchets, comprenant :
- un réceptacle (9 ; 62) doté d'une ouverture (10 ; 63) d'introduction des déchets,
- une gaine (19) s'étendant à l'intérieur du réceptacle (9 ; 62) au travers de l'ouverture (10 ; 63)
- un obstacle de changement de direction (9a ; 66, 67) disposé en aval de l'ouverture d'introduction (10 ; 63), transversalement par rapport à la gaine (19), ledit obstacle étant agencé pour se trouver sur le trajet de ladite gaine de façon à incurver la trajectoire de celle-ci,
- la gaine (19) étant disposée autour du réceptacle (9 ; 62) à l'extérieur de ce dernier, et s'invagine à l'intérieur dudit réceptacle au travers de l'ouverture (10 ; 63), ladite gaine présentant une section inférieure à celle du réceptacle (9) et étant apte à s expanser radialement,
ledit dispositif étant caractérisé en ce que :
- des moyens d'entraînement (26-32, 34-37 ; 73-82, 86-90) de la gaine (19) sont adaptés pour coopérer avec un tronçon de celle-ci se trouvant en aval de l'obstacle de changement de direction et pour :
. maintenir constamment en tension la portion de gaine (19) située en amont desdits moyens d'entraînement de façon à provoquer une fermeture de ladite gaine au contact de l'obstacle de changement de direction (9a ; 66, 67),
. entraîner, à la commande, un déplacement de l'ensemble de la gaine (19) sur une distance prédéterminée, de façon à amener un nouveau tronçon de gaine à pénétrer dans le réceptacle (9 ; 62),
- des moyens d'actionnement (33 ; 54) sont associés aux moyens d'entraînement,
- et une zone de stockage de la gaine (19) est disposée en aval des moyens d'entraînement (26-32, 34-37 ; 73-82, 86-90).

2. Dispositif selon la revendication 1, caractérisé en ce que le réceptacle (9 ; 62) présente une section tubulaire présentant une section croissante s'évasant en direction de l'ouverture d'introduction (10 ; 63).

3. Dispositif selon l'une des revendications 1 ou 2, caractérisé en ce qu'il comprend :
- un dévidoir (20 ; 71) de section tubulaire disposé entre l'obstacle de changement de direction (9a ; 66, 67) et les moyens d'entraînement (26-32, 34-37 ; 73-82, 86-90), et agencé pour que la gaine (19) passe à l'intérieur dudit dévidoir,
- une deuxième gaine étanche (25) stockée autour du dévidoir (20 ; 71) à l'extérieur de ce dernier et adaptée pour se dévider autour de la première gaine (19), de façon à ensacher cette dernière et à être entraînée avec celle-ci par les moyens d'entraînement (26-32, 34-37 ; 73-82, 86-90).

4. Dispositif selon l'une des revendications précédentes caractérisé en ce que l'obstacle de changement de direction est constitué d'un coude (9a) disposé dans le prolongement de l'ouverture (10) et agencé pour que la gaine (19) se referme au contact de la portion supérieure dudit coude.

5. Dispositif selon l'une des revendications 1 à 3, caractérisé en ce que l'obstacle de changement de direction est constitué de deux axes parallèles (66, 67) dits amont et aval, disposés transversalement a distance l'un de l'autre de façon que lors du passage de la gaine (19) entre lesdits axes, la trajectoire de cette dernière soit incurvée de telle manière que ladite gaine se referme au contact de l'axe aval (67).

6. Dispositif selon l'une des revendications précédentes, caractérisé en ce que les moyens d'entraînement comprennent :
- des moyens de traction (26-32, 34-37) adaptés pour entraîner un déplacement de la gaine (19) sur une distance prédéterminée, de façon à amener un nouveau tronçon de gaine à pénétrer dans le réceptacle (9),
- des moyens de stockage situés dans la zone de stockage et comportant une bobine d'envidage (39) portée par un arbre de rotation (40), et des moyens (42) d'entraînement en rotation de ladite bobine aptes à la faire tourner d'une distance angulaire équivalant à la distance linéaire de déplacement de la gaine (19), lors de chaque actionnement des moyens de traction (26-32, 34-37).

7. Dispositif selon la revendication 6, caractérisé en ce que les moyens de traction comprennent :
- deux rouleaux transversaux (26, 27) disposés de façon à se trouver de part et d'autre de la gaine (19), lesdits rouleaux étant solidaires, au niveau de chacune de leurs extrémités, d'un axe (28) excentré par rapport à leur axe de symétrie longitudinal,
- des moyens d'entraînement (32) des rouleaux (26, 27), commandés par les moyens d'actionnement (33) et aptes à déplacer ensemble lesdits rouleaux le long de la gaine (19) entre une position amont et une position aval,
- des moyens de rappel (34) aptes à ramener les rouleaux (26, 27) de leur position aval vers leur position amont,
- des moyens de pivotement (35-37) des axes (28) des rouleaux (26, 27) aptes à maintenir lesdits rouleaux :
. en contact l'un contre l'autre lors de leur déplacement de leur position amont vers leur position aval, de façon à entraîner la gaine (19) pincée entre lesdits rouleaux,
. éloignés l'un de l'autre lors de leur retour de leur position aval vers leur position amont.

8. Dispositif selon la revendication 7, caractérisé en ce que :
- les moyens de pivotement comprennent, pour chaque rouleau (26, 27), un levier (35) solidaire de l'axe (28) dudit rouleau, et des organes de butée amont (37) et aval (36) aptes à entraîner la rotation dudit levier,
- des moyens élastiques bistables (38) sont associés aux leviers (35) et agencés pour maintenir les rouleaux (26, 27) dans l'une ou l'autre de leur position relative, après pivotement desdits leviers.

9. Dispositif selon l'une des revendications 7 ou 8, caractérisé en ce que les moyens d'entraînement en rotation de la bobine (39) comprennent un moteur ressort (42) précontraint dans une phase préalable et adapté pour provoquer une rotation de ladite bobine lorsque les rouleaux (26, 27) sont dans leur position éloignés l'un de l'autre.

10. Dispositif selon les revendications 4 et 6 prises ensemble, caractérisé en ce qu'il comprend un carter (8) doté d'une face supérieure ouverte, apte à loger le réceptacle (9), les moyens de traction (26-32, 34-37) et les moyens de stockage (39), lesdits réceptacle et moyens de stockage étant montés de façon amovible à l'intérieur dudit carter.

11. Dispositif selon la revendication 10, caractérisé en ce que :
- le réceptacle (9) comporte deux axes transversaux (11, 12), fixés extérieurement sur et sous ledit réceptacle vers son extrémité opposée à l'ouverture d'introduction (10),
- le carter (8) comporte quatre glissières (13, 17) agencées pour recevoir à coulissement les axes (11, 12) du réceptacle (9), et dotées, vers leur extrémité, d'organes (14, 18) de blocage en translation desdits axes.

12. Dispositif selon les revendications 3 et 11 prises ensemble, caractérisé en ce que :
- la gaine étanche (25) est stockée autour du dévidoir (20) de façon à s'invaginer à l'intérieur dudit dévidoir, autour de la première gaine (19),
- le dévidoir (20) est adapté pour venir se loger sur sa plus grande longueur dans le réceptacle (9), en aval du coude, et comporte deux axes transversaux (21, 22) fixés extérieurement sur et sous ledit dévidoir, vers une des extrémités de ce dernier, lesdits axes, de longueur inférieure aux axes (11, 12) du réceptacle (9) étant portés au niveau de chacune de leurs extrémités par une pièce amovible (23, 24) de liaison avec un axe (11, 12) du réceptacle (9).

13. Dispositif selon l'une des revendications 10 à 12, caractérisé en ce que le carter (8) comporte deux gorges (41) inclinées par rapport à la verticale aptes à loger les extrémités de l'arbre de rotation (40) de la bobine (39).

14. Dispositif selon l'une des revendications 10 à 13, caractérisé en ce qu'il comprend un chariot (1-3) apte à porter le carter (8), doté d'un habillage (4) comportant un capot amovible (6) percé d'une ouverture (7) conjuguée de l'ouverture (10) d'introduction du réceptacle (9).

15. Dispositif selon la revendication 14, caractérisé en ce qu'il comprend des moyens d'aspiration aptes à créer une dépression à l'intérieur de l'habillage, et des moyens de filtration de l'air refoulé par lesdits moyens d'aspiration.

16. Dispositif selon l'une des revendications 1 à 5, caractérisé en ce que les moyens d'entraînement de la gaine (19) comprennent :
- deux rouleaux transversaux (73, 74) portés chacun par un arbre de rotation (75, 76), disposés de part et d'autre de la gaine (19) de façon que celle-ci soit maintenue pincée entre lesdits rouleaux,
- des moyens (79-82, 86-90) d'entraînement en rotation des rouleaux (73, 74), commandés par les moyens d'actionnement (54), aptes à chaque commande, à faire tourner lesdits rouleaux de façon unidirectionnelle et en sens inverse, d'une distance angulaire prédéterminée,
- des moyens de basculement (77) de l'arbre de rotation (76) d'au moins un des rouleaux (74), aptes à permettre aux rouleaux (73, 74) de s'écarter l'un de l'autre,
- et des moyens de rappel (78) reliant les arbres de rotation (75, 76) des rouleaux (73, 74), de façon à exercer une force tendant à maintenir ces derniers en contact l'un contre l'autre.

17. Dispositif selon la revendication 16, caractérise en ce que les rouleaux (73, 74) comportent une face périphérique présentant des cannelures s'étendant selon les génératrices desdits rouleaux.

18. Dispositif selon l'une des revendications 16 ou 17, caractérisé en ce que :
- l'arbre de rotation (75) d'un des rouleaux (73), dit fixe, est monté fixe en translation, l'arbre (76) de l'autre rouleau (74), dit mobile, étant solidaire de leviers (77) aptes à permettre un basculement dudit rouleau mobile par rapport au rouleau fixe (73),
- les moyens d'entraînement en rotation des rouleaux comprennent des moyens (79-82, 86) d'entraînement unidirectionnel du rouleau fixe (73), associés aux moyens d'actionnement (54), et des organes de transmission (87-90) aptes à transmettre ce mouvement de rotation, de façon inversée, au rouleau mobile (74).

19. Dispositif selon la revendication 18, caractérisé en ce que les organes de transmission comprennent :
- une poulie à deux gorges (87) montée de façon coaxiale par rapport à l'axe de rotation des leviers (77),
- deux poulies (88) solidaires chacune de l'arbre de rotation (75, 76) d'un des rouleaux (73, 74),
- deux courroies (89, 90) reliant chacune la poulie (88) d'un des rouleaux (73, 74) à la poulie à deux gorges (87), et disposées de façon à inverser le sens de rotation du rouleau mobile (74) par rapport à celui du rouleau fixe (73).

20. Dispositif selon la revendication 5 et l'une des revendications 16 à 19, caractérisé en ce que le réceptacle (62), les moyens d'entraînement (73-82, 86-90) de la gaine (19) et la zone de stockage sont agencés les uns en dessous des autres.

21. Dispositif selon la revendication 20, caractérisé en ce qu'il comprend une coque (55) dotée d'une ouverture supérieure (58), apte à loger le réceptacle (62) et les moyens d'entraînement (73-82, 86-90), et à délimiter dans sa partie inférieure une zone de stockage accessible, le réceptacle (62) étant monté de façon amovible dans ladite coque.

22. Dispositif selon les revendications 3 et 21 prises ensemble, caractérisé en ce que :
- la gaine étanche (25) est stockée autour du dévidoir (71) de façon à se dévider dans le prolongement inférieur dudit dévidoir, autour de la première gaine (19) sortant de ce dernier,
- le dévidoir (71) présente une collerette supérieure (72) agencée pour permettre de suspendre ledit dévidoir à l'intérieur de la coque (55) dotée à cet effet d'éléments supports (91) de ladite collerette,
- le réceptacle (62) est agencé pour venir reposer sur la collerette (72) du dévidoir (71).

23. Dispositif selon la revendication 22, caractérisé en ce que le dévidoir (71) présente une section tubulaire rectangulaire, et comporte deux faces latérales (71a) présentant longitudinalement une forme concave.

24. Dispositif selon l'une des revendications 20 à 23, caractérisé en ce que :
- le réceptacle (62) présente une section tubulaire ovoïde et comporte une portion supérieure (62a) de section croissante s'évasant en direction de l'ouverture d'introduction (63), et une portion inférieure (62b) de section constante,
- les axes (66, 67) s'étendent selon la plus grande dimension du réceptacle (62), l'axe amont (66) étant disposé dans la portion supérieure (62a) dudit réceptacle, et l'axe aval (67) étant disposé dans la portion inférieure (62b) et étant relié à l'axe amont (66) de façon à pouvoir basculer relativement à ce dernier.

25. Dispositif selon la revendication 24, caractérisé en ce que l'axe amont (66) est entouré d'un tube (68), l'axe aval (67) consistant en l'âme d'un U dont les extrémités des ailes (69) sont solidaires dudit tube.

26. Dispositif selon l'une des revendications précédentes, caractérisé en ce qu'il comprend une pompe (51) apte à être commandée par les moyens d'actionnement (33) et dotée d'une entrée d'aspiration reliée à un réservoir (52) destiné à renfermer un produit antiseptique, et une sortie de refoulement reliée à un injecteur (53) disposé dans le réceptacle (9).

27. Dispositif selon les revendications 1, 3 et 4 prises ensemble, destiné à servir de cabinet d aisances, caractérisé en ce que :
- le réceptacle (9) est conformé, en aval du coude, de façon à constituer un bac (43) de récupération de liquides, doté d'une sortie d'évacuation desdits liquides,
- la première gaine (19) présente une texture adaptée pour laisser s'écouler les liquides au travers de ladite gaine vers le bac de récupération (43),
- des moyens de traitement (45-47) sont adaptés pour assurer le traitement des liquides récupérés en vue de permettre leur rejet et/ou leur utilisation.

28. Dispositif selon la revendication 27, caractérisé en ce que les moyens de traitement comprennent des moyens (45) de centrifugation des liquides, des moyens (46) de pressurisation des liquides récupérés après centrifugation, et des moyens (47) de filtration vers lesquels les liquides sont délivrés sous pression.

29. Dispositif selon l'une des revendications 27 ou 28, caractérisé en ce qu'il est logé dans une enceinte comportant des moyens d'aspiration (48) aptes à créer une dépression à l'intérieur de ladite enceinte, et des moyens (49, 50) de filtration de l'air refoulé par lesdits moyens d'aspiration.

## Claims

1. Waste-recovery device comprising:
- a receiving unit (9 ; 62) having an opening (10 ; 63) for feeding in waste,
- a sheath (19) extending into the receiving unit (9 ; 62) through the opening (10 ; 63),
- a deflecting and blocking unit (9a ; 66, 67) located transverse to the sheath (19) downstream of the feed opening (10 ; 63), said blocking unit being so arranged as to be situated in the path of said sheath in such a way as to deflect the trajectory of the latter inward,
- whereby the sheath (19) is disposed around the receiving unit (9 ; 62) outside the latter and is introduced into said receiving unit through the opening (10 ; 63), said sheath having a cross-section smaller than that of the receiving unit (9) and being capable of radial expansion,
said device being characterised in that:
- entraining means (26-32, 34-37 ; 73-82, 86-90) for the sheath (19) are designed to cooperate with a section of the latter located downstream of the deflecting and blocking unit as well as:
. to maintain that part of the sheath (19) situated upstream of said entraining means constantly in a state of tension so as to cause said sheath to be closed off when it contacts the deflecting and blocking unit (9a ; 66, 67)
. to bring about, on command, a displacement of the entire sheath (19) by a predetermined distance so as to cause a fresh section of the sheath to enter the receiving unit (9 ; 62),
- actuating means (33 ; 54) are associated with the entraining means,
- and a storage zone for the sheath (19) is located downstream of the entraining means (26-32, 34-37 ; 73-82, 86-90).

2. Device according to Claim 1, characterised in that the receiving unit (9 ; 62) has a tubular cross-section, said cross-section increasing as it flares outward in the direction of the feed opening (10 ; 63).

3. Device according to Claim 1 or Claim 2, characterised in that it comprises:
- an unwinding unit (20 ; 71) having a tubular cross-section located between the deflecting and blocking unit (9a ; 66, 67) and the entraining means (26-32, 34-37 ; 73-82, 86-90) and so arranged as to ensure that the sheath (19) passes into said unwinding unit
- a second impermeable sheath (25) stored around the unwinding unit (20 ; 71) outside the latter and so designed as to be unwound around the first sheath (19) in such a way as to encase said first sheath and to be entrained jointly with the latter by the entraining means (26-32, 34-37 ; 73-82, 86-90).

4. Device according to one of the preceding claims, characterised in that the deflecting and blocking unit is constituted by a bend (9a) located in the extension of the opening (10) and so arranged as to ensure that the sheath (19) is closed off when it contacts the upper part of said bend.

5. Device according to one of Claims 1 to 3, characterised in that the deflecting and blocking unit is constituted by two parallel spindles (66, 67), the so-called upstream spindle and the so-called downstream spindle, said spindles being located transversely at a distance from one another so as to ensure that the path of the sheath (19) as the latter passes between said spindles is deflected inward in such a way that said sheath is closed off when it contacts the downstream spindle (67).

6. Device according to one of the preceding claims, characterised in that the entraining means comprise:
- traction means (26-32, 34-37) so designed as to bring about a displacement of the sheath (19) by a predetermined distance so as to cause a fresh section of the sheath to enter the receiving unit (9),
- storage means situated within the storage zone and comprising a take-up reel (39) mounted on a rotary shaft (40) as well as means (42) for driving said reel in rotation, said driving means being so designed as to cause said reel to rotate by an angular distance equivalent to the linear distance by which the sheath (19) is displaced whenever the traction means (26-32, 34-37) are actuated.

7. Device according to Claim 6, characterised in that the traction means comprise:
- two transverse rollers (26, 27) so disposed as to be on either side of the sheath (19), said rollers being firmly secured, in the region of each of their ends, to a spindle (28) which is offset in relation to their longitudinal axis of symmetry,
- driving means (32) for the rollers (26, 27), these driving means being controlled with the aid of the actuating means (33) and capable of jointly displacing said rollers along the sheath (19) between an upstream position and a downstream position,
- resetting means (34) capable of returning the rollers (26, 27) from their downstream position to their upstream position,
- pivoting means (35-37) for the spindles (28) of the rollers (26, 27), said pivoting means being capable of maintaining said rollers:
. in contact with one another when they are displaced from their upstream position to their downstream position so as to entrain the sheath (19) which is clamped between said rollers,
. at a distance from one another when they return from their downstream position to their upstream position.

8. Device according to Claim 7 characterised in that:
- the pivoting means comprise, for each roller (26, 27), a lever (35) which is firmly secured to the spindle (28) of said roller as well as stop elements, i.e. an upstream stop element (37) and a downstream stop element (36), capable of passing on the rotation of said lever,
- bistable elastic means (38), said means being associated with the levers (35) and so arranged as to maintain, after pivoting said levers, the rollers (26, 27) in one or the other of their relative positions.

9. Device according to Claim 7 or Claim 8, characterised in that the means for driving the reel (39) in rotation comprise a spring motor (42) prestressed at a previous stage and so designed as to bring about rotation of said reel when the rollers (26, 27) are in the position in which they are at a distance from one another.

10. Device according to Claims 4 and 6 jointly, characterised in that it comprises a case (8) having an open upper side and so designed as to be capable of accommodating the receiving unit (9), the traction means (26-32, 34-37) and the storage means (39), said receiving units and said storage means being mounted in removable manner within said case.

11. Device according to Claim 10, characterised in that:
- the receiving unit (9) comprises two transverse spindles (11, 12) mounted externally above and below said receiving unit near its end opposite the feed opening (10),
- the case (8) comprises four guide rails (13, 17) arranged to accommodate in sliding manner the spindles (11, 12) of the receiving unit (9) and provided near their ends with elements (14, 18) for blocking the translational motion of said spindles.

12. Device according to Claims 3 and 11 jointly, characterised in that:
- the impervious sheath (25) is stored around the unwinding unit (20) so as to be introduced into said unwinding unit around the first sheath (19),
- the unwinding unit (20) is so designed that the greater part of its length comes to be located within the receiving unit (9) downstream of the bend and comprises two transverse spindles (21, 22) mounted externally above and below said unwinding unit near one of the ends of the latter, said spindles, the length of which is smaller than that of the spindles (11, 12) of the receiving unit (9), being supported at each of their ends by a removable element (23, 24) for connection to a spindle (11, 12) of the receiving unit (9).

13. Device according to one of Claims 10 to 12, characterised in that the case (8) comprises two slots (41) which are inclined in relation to the vertical and capable of accommodating the ends of the rotary shaft (40) of the reel (39).

14. Device according to one of Claims 10 to 13, characterised in that it comprises a carriage (1-3) capable of supporting the case (8), said carriage being provided with a casing (4) comprising a removable cover (6) with an opening (7) which matches the feed opening (10) of the receiving unit (9).

15. Device according to Claim 14, characterised in that it comprises suction means capable of creating a partial vacuum within the casing as well as means for filtering the air delivered by said suction means.

16. Device according to one of Claims 1 to 5, characterised in that the means for entraining the sheath (19) comprise:
- two transverse rollers (73, 74) each of which is mounted on a rotary shaft (75, 76), said rollers being located on either side of the sheath (19) so that the latter is kept clamped between said rollers,
- means (79-82, 86-90) for driving the rollers (73, 74) in rotation, said means being controlled by the actuating means (54) and capable of causing, on each command, said rollers to rotate in unidirectional manner and in inverse direction by a predetermined angular distance,
- means (77) for tilting the rotary shaft (76) of at least one of the rollers (74), said means being capable of enabling the rollers (73, 74) to move apart from one another,
- and resetting means (78) connecting the rotary shafts (75, 76) of the rollers (73, 74) so as to exert a force tending to maintain the latter in contact with one another.

17. Device according to Claim 16, characterised in that the rollers (73, 74) comprise a peripheral face with grooves extending along the generating lines of said rollers.

18. Device according to Claim 16 or 17, characterised in that:
- the rotary shaft (75) of one of the rollers (73), the so-called fixed roller, is mounted so as to be incapable of translational motion whereas the shaft (76) of the other roller (74), the so-called mobile roller, is firmly secured to levers (77), said levers being capable of enabling tilting of said mobile roller in relation to the fixed roller (73),
- the means for driving the rollers in rotation comprise means (79-82, 86) for unidirectional driving of the fixed roller (73), said means being associated with the actuating means (54), as well as transmission elements (87-90) capable of transmitting said rotary motion in inverse direction to the mobile roller (74).

19. Device according to Claim 18, characterised in that the transmission elements comprise:
- a twin-grooved pulley (87) coaxially mounted in relation to the axis of rotation of the levers (77),
- two pulleys (88) each of which is firmly secured to the rotary shaft (75, 76) of one of the rollers (73, 74),
- two belts (89, 90) each connecting the pulley (88) of one of the rollers (73, 74) to the twin-grooved pulley (87) and so arranged as to cause the direction in which the mobile roller (74) rotates to be opposite to the direction in which the fixed roller (73) rotates.

20. Device according to Claim 5 and one of Claims 16 to 19, characterised in that the receiving unit (62), the entraining means (73-82, 86-90) for the sheath (19) and the storage zone are arranged below one another.

21. Device according to Claim 20, characterised in that it comprises a shell (55) provided with an upper opening (58), said shell being capable of accommodating the receiving unit (62) and the entraining means (73-82, 86-90) as well as of delimiting in its lower part an accessible storage zone, the receiving unit (62) being mounted in removable manner within said shell.

22. Device according to Claims 3 and 21 jointly, characterised in that:
- the impervious sheath (25) is stored around the unwinding unit (71) so as to be unwound in the lower extension of said unwinding unit around the first sheath (19) which comes out from the unwinding unit,
- the unwinding unit (71) is provided with an upper collar (72) so arranged as to enable said unwinding unit to be suspended within the shell (55), the latter being provided to this end with supporting elements (91) for said collar,
- the receiving unit (62) is so arranged as to be seated on the collar (72) of the unwinding unit (71).

23. Device according to Claim 22, characterised in that the unwinding unit (71) has a rectangular tubular cross-section and comprises two side faces (71a), the longitudinal shape of which is concave.

24. Device according to one of Claims 20 to 23, characterised in that:
- the receiving unit (62) has an ovoid tubular cross-section and comprises an upper part (62a), the cross-section of which increases as it flares outward in the direction of the feed opening (63), as well as a lower part (62b) having a constant cross-section,
- the spindles (66, 67) extend in the lengthwise direction of the receiving unit (62), the upstream spindle (66) being arranged within the upper part (62a) of said receiving unit and the downstream spindle (67) being arranged within the lower part (62b) of said unit and so connected to the upstream spindle (66) as to be capable of tilting in relation to the latter.

25. Device according to Claim 24, characterised in that the upstream spindle (66) is surrounded by a tube (68) whereas the downstream spindle (67) consists of the connecting cross bar of a U, the ends of the lateral legs (69) being firmly secured to said tube.

26. Device according to one of the preceding claims, characterised in that it comprises a pump (51) capable of being controlled by the actuating means (33) and provided with a suction inlet connected to a reservoir (52) intended for use as a container for an antiseptic product and also comprises a discharge outlet which is connected to an injector (53) arranged within the receiving unit (9).

27. Device according to Claims 1, 3 and 4 jointly for use as a lavatory, characterised in that:
- downstream of the bend the receiving unit (9) is so designed as to constitute a liquid-recovery sump (43) provided with an outlet for evacuating said liquids,
- the first sheath (19) is structured in such a way as to allow the liquids to flow through said sheath towards the recovery vessel (43),
- processing means (45-47) are so designed as to ensure processing of the liquids recovered in order to enable their disposal and/or their utilisation.

28. Device according to Claim 27, characterised in that the processing means comprise means (45) for centrifuging liquids, means (46) for pressurising the liquids recovered after centrifugation, and filtering means (47) towards which the liquids are delivered under pressure.

29. Device according to Claim 27 or Claim 28, characterised in that it is installed within an enclosure comprising suction means (48) capable of creating a partial vacuum within said enclosure as well as means (49, 50) for filtering the air delivered by said suction means.

## Patentansprüche

1. Vorrichtung zum Erfassen von Abfällen, umfassend:
- eine Aufnahmeeinheit (9 ; 62) mit einer Öffnung (10 ; 63) zum Einführen von Abfällen,
- eine Hülle (19), die sich durch die Öffnung (10 ; 63) hindurch in das Innere der Aufnahmeeinheit (9 ; 62) erstreckt,
- einen stromabwärts von der Einführöffnung (10 ; 63) im Verhältnis zu der Hülle (19) quer angeordneten Umlenksperrmechanismus (9a ; 66, 67), wobei sich der besagte Sperrmechanismus infolge seiner Anordnung in der Bahn der besagten Hülle befindet, so daß er den Verlauf der besagten Hülle in Einwärtsrichtung umlenkt,
- wobei die rings um die Aufnahmeeinheit (9 ; 62) außerhalb dieser angeordnete Hülle (19) durch die Öffnung (10 ; 63) hindurch in das Innere der besagten Aufnahmeeinheit eingezogen wird und der Querschnitt der besagten Hülle geringer als der Querschnitt der Aufnahmeeinheit (9) und zu radialer Ausdehnung in der Lage ist,
und zwar ist die besagte Vorrichtung dadurch gekennzeichnet,
- daß Einzugsmittel (26-32, 34-37 ; 73-82, 86-90) für die Hülle (19) so beschaffen sind, daß sie mit einem stromabwärts von dem Umlenksperrmechanismus befindlichen Abschnitt der besagten Hülle zusammenwirken und:
. daß der stromaufwärts von den besagten Einzugsmitteln befindliche Teil der Hülle (19) laufend in gespanntem Zustand erhalten wird, um Verschluß der besagten Hülle zu bewirken, wenn diese mit dem Umlenksperrmechanismus (9a ; 66, 67) in Kontakt kommt,
. daß auf Anweisung eine Verlagerung der gesamten Hülle (19) um eine vorbestimmte Strecke bewirkt wird, um einen frischen Abschnitt der Hülle in die Aufnahmeeinheit (9 ; 62) einzuziehen,
- daß Betätigungsmittel (33 ; 54) mit den Einzugsmitteln in Verbindung stehen,
- und daß ein Bereich zur Lagerung der Hülle (19) stromabwärts von den Einzugsmitteln (26-32, 34-37 ; 73-82, 86-90) angeordnet ist.

2. Vorrichtung nach Anspruch 1, dadurch gekennzeichnet, daß die Aufnahmeeinheit (9 ; 62) einen rohrförmigen Querschnitt aufweist, wobei die Größe des besagten sich in Richtung der Einführöffnung (10 ; 63) aufweitenden Querschnitts zunimmt.

3. Vorrichtung nach Anspruch 1 oder Anspruch 2, dadurch gekennzeichnet, daß sie folgende Teile umfaßt:
- eine Abrolleinheit (20 ; 71) mit rohrförmigem Querschnitt, wobei die besagte Abrolleinheit zwischen der Umlenksperrmechanismus (9a ; 66, 67) und den Einzugsmitteln (26-32, 34-37 ; 73-82, 86-90) vorgesehen und so angeordnet ist, daß die Hülle (19) in das Innere der besagten Abrolleinheit eindringt,
- eine zweite undurchlässige Hülle (25), die rings um die Abrolleinheit (20 ; 71) außerhalb dieser gelagert und so beschaffen ist, daß sie rings um die erste Hülle (19) abgewickelt wird, wobei sie diese umschließt, und daß sie durch Einzugsmittel (26-32, 34-37 ; 73-82, 86-90) gemeinsam mit der besagten ersten Hülle eingezogen wird.

4. Vorrichtung nach einem der vorstehenden Ansprüche, dadurch gekennzeichnet, daß der Umlenksperrmechanismus aus einem Krümmer (9a) besteht, der in der Verlängerung der Öffnung (10) vorgesehen und so angeordnet ist, daß sich die Hülle (19), indem sie mit dem oberen Teil des besagten Krümmers in Kontakt kommt, schließt.

5. Vorrichtung nach einem der Ansprüche 1 bis 3, dadurch gekennzeichnet, daß sich der Umlenksperrmechanismus aus zwei parallelen Spindeln (66, 67), der sogenannten stromaufwärts befindlichen Spindel und der sogenannten stromabwärts befindlichen Spindel, zusammensetzt, die in einem Abstand voneinander quer angeordnet sind, so daß während des Durchgangs der Hülle (19) zwischen den besagten Spindeln der Verlauf der besagten Hülle so in Einwärtsrichtung umgelenkt wird, daß sich die besagte Hülle, wenn sie mit der stromabwärts befindlichen Spindel (67) in Kontakt kommt, schließt.

6. Vorrichtung nach einem der vorstehenden Ansprüche, dadurch gekennzeichnet, daß die Einzugsmittel folgende Teile umfassen:
- Zugmittel (26-32, 34-37), die so beschaffen sind, daß sie eine Verlagerung der Hülle (19) um eine vorbestimmte Strecke bewirken, um einen frischen Abschnitt der Hülle in die Aufnahmeeinheit (9) einzuziehen,
- in dem Lagerbereich befindliche und eine auf einer Drehwelle (40) angeordnete Aufwickelspule (39) umfassende Lagermittel sowie Mittel (42) für drehenden Antrieb der besagten Spule, die in der Lage sind, die besagte Spule um einen der linearen Verlagerungsstrecke der Hülle (19) äquivalenten Winkelabstand zu drehen, wenn immer die Zugmittel (26-32, 34-37) betätigt werden.

7. Vorrichtung nach Anspruch 6, dadurch gekennzeichnet, daß die Zugmittel folgende Teile umfassen:
- zwei Querwalzen (26, 27), die so angeordnet sind, daß sie sich zu beiden Seiten der Hülle (19) befinden, wobei die besagten Walzen im Bereiche jedes ihrer Enden fest mit einer im Verhältnis zu ihrer Symmetrielängsachse exzentrischen Spindel (28) verbunden sind,
- Antriebsmittel (32) für die Walzen (26, 27), wobei die besagten Antriebsmittel durch die Betätigungsmittel (33) gesteuert werden und in der Lage sind, die besagten Walzen gemeinsam entlang der Hülle (19) zwischen einer Stromaufwärtsposition und einer Stromabwärtsposition zu verlagern,
- Rückstellmittel (34), die in der Lage sind, die Walzen (26, 27) aus ihrer Stromabwärtsposition in ihre Stromaufwärtsposition zurückzuführen,
- Schwenkmittel (35-37) für die Spindeln (28) der Walzen (26, 27), wobei die besagten Schwenkmittel in der Lage sind, die besagten Walzen:
. während deren Verlagerung aus ihrer Stromaufwärtsposition in ihre Stromabwärtsposition miteinander in Kontakt zu erhalten, um die zwischen den besagten Walzen geklemmte Hülle (19) einzuziehen,
. die besagten Walzen während ihrer Rückführung aus der Stromabwärtsposition in die Stromaufwärtsposition in Abstand voneinander zu erhalten.

8. Vorrichtung nach Anspruch 7, dadurch gekennzeichnet:
- daß die Schwenkmittel für jede Walze (26, 27) einen Hebel (35) umfassen, der mit der Spindel (28) der besagten Welle fest verbunden ist, sowie Anschlagelemente, ein stromaufwärts befindliches Anschlagelement (37) und ein stromabwärts befindliches Anschlagelement (36), die in der Lage sind, die Drehung des besagten Hebels zu übertragen,
- daß bistabile elastische Mittel (38) mit den Hebeln (35) in Verbindung stehen und so angeordnet sind, daß sie die Walzen (26, 27) nach dem Schwenken der besagten Hebel in der einen oder anderen ihrer relativen Positionen erhalten.

9. Vorrichtung nach Anspruch 7 oder Anspruch 8, dadurch gekennzeichnet, daß die Mittel für drehenden Antrieb der Spule (39) einen Federmotor (42) umfassen, der in einer vorhergehenden Phase vorgespannt wird und so beschaffen ist, daß er, wenn die Walzen (26, 27) in der Position, in der sie voneinander entfernt sind, Drehung der besagten Spule bewirkt.

10. Vorrichtung nach den Ansprüchen 4 und 6 gemeinsam, dadurch gekennzeichnet, daß sie ein eine offene Oberseite aufweisendes Gehäuse (8) umfaßt, das geeignet ist, die Aufnahmeeinheit (9), die Zugmittel (26-32, 34-37) und die Lagermittel (39) unterzubringen, wobei die besagte Aufnahmeeinheit und die besagten Lagermittel auf abmontierbare Weise im Inneren des besagten Gehäuses angeordnet sind.

11. Vorrichtung nach Anspruch 10, dadurch gekennzeichnet:
- daß die Aufnahmeeinheit (9) zwei Querspindeln (11, 12) umfaßt, die extern oberhalb bzw. unterhalb der besagten Aufnahmeeinheit im Bereich ihres der Einführöffnung (10) gegenüber liegenden Endes angebracht sind,
- daß das Gehäuse (8) vier Führungsschienen (13, 17) umfaßt, die so angeordnet sind, daß sie die Spindeln (11, 12) der Aufnahmeeinheit (9) gleitend aufnehmen, und die im Bereiche ihrer Enden Elemente (14, 18) zum Sperren von Translationsbewegungen der besagten Spindeln aufweisen.

12. Vorrichtung nach den Ansprüchen 3 und 11 gemeinsam, dadurch gekennzeichnet:
- daß die undurchlässige Hülle (25) so rings um die Abrolleinheit (20) gelagert ist, daß sie die erste Hülle (19) umschließend in das Innere der besagten Abrolleinheit eingezogen wird,
- daß die Abrolleinheit (20) so beschaffen ist, daß sie mit dem größeren Teil ihrer Länge stromabwärts von dem Krümmer in der besagten Aufnahmeeinheit (9) zu liegen kommt und zwei Querspindeln (21, 22) umfaßt, die extern oberhalb bzw. unterhalb der besagten Abrolleinheit im Bereiche eines von deren Enden angebracht sind, wobei die besagten Spindeln, deren Länge geringer ist als die der Spindeln (11, 12) der Aufnahmeeinheit (9) im Bereiche jedes ihrer Enden durch ein abmontierbares Element (23, 24) zur Verbindung mit einer Spindel (11, 12) der Aufnahmeeinheit (9) abgestützt sind.

13. Vorrichtung nach einem der Ansprüche 10 bis 12, dadurch gekennzeichnet, daß das Gehäuse (8) zwei im Verhältnis zu der Senkrechten geneigte Schlitze (41) umfaßt, die in der Lage sind, die Enden der Drehwelle (40) der Spule (39) aufzunehmen.

14. Vorrichtung nach einem der Ansprüche 10 bis 13, dadurch gekennzeichnet, daß sie ein Fahrgestell (1-3) umfaßt, das in der Lage ist, das Gehäuse (8) abzustützen, wobei das besagte Fahrgestell eine Umkleidung (4) mit einem entfernbaren Deckel (6) umfaßt, der eine der Einführöffnung (10) der Aufnahmeeinheit (9) entsprechende Öffnung (7) aufweist.

15. Vorrichtung nach Anspruch 14, dadurch gekennzeichnet, daß sie Saugmittel umfaßt, die zur Erzeugung eines Unterdrucks im Inneren der Verkleidung in der Lage sind, sowie Mittel zur Filtration der durch die besagten Saugmittel geförderten Luft.

16. Vorrichtung nach einem der Ansprüche 1 bis 5, dadurch gekennzeichnet, daß die Einzugsmittel für die Hülle (19) folgende Teile umfassen:
- zwei Querwalzen (73, 74), die jeweils auf einer Drehwelle (75, 76) angeordnet sind und sich auf der einen und auf der anderen Seite der Hülle (19) befinden, so daß die besagte Hülle in dem Zustand, in dem sie zwischen den besagten Walzen geklemmt ist, erhalten wird,
- Mittel (79-82, 86-90) für drehenden Antrieb der Walzen (73, 74), wobei die besagten Antriebsmittel durch die Betätigungsmittel (54), die auf jede Anweisung in der Lage sind, Drehung der besagten Walzen um einen vorbestimmten Winkelabstand in einer Richtung sowie in der entgegengesetzten Richtung zu bewirken, gesteuert werden,
- Mittel (77) zum Verschwenken der Drehwelle (76) mindestens einer der Walzen (74), wobei diese so beschaffen sind, daß sich die Walzen (73, 74) voneinander entfernen können,
- sowie Rückstellmittel (78), die die Drehwellen (75, 76) der Walzen (73, 74) so miteinander verbinden, daß sie eine Kraft ausüben, die die Tendenz hat, die besagten Walzen miteinander in Kontakt zu erhalten.

17. Vorrichtung nach Anspruch 16, dadurch gekennzeichnet, daß die Walzen (73, 74) eine Umfangsfläche mit Rillen aufweisen, die sich entlang den Erzeugenden der besagten Walzen erstrecken.

18. Vorrichtung nach Anspruch 16 oder Anspruch 17, dadurch gekennzeichnet:
- daß die Drehwelle (75) einer der Walzen (73), der sogenannten feststehenden Walze, in Translationshinsicht fest angeordnet und die Welle (76) der anderen Walze (74), der sogenannten mobilen Walze, mit Hebeln (77) fest verbunden ist, wobei diese so beschaffen sind, daß sie Verschwenkung der besagten mobilen Walze im Verhältnis zu der feststehenden Walze (73) gestatten,
- daß die Mittel für drehenden Antrieb der Walzen mit den Betätigungsmitteln (54) in Verbindung stehende Mittel (79- 82, 86) zum Antrieb der feststehenden Walze (73) in einer Richtung umfassen, sowie Übertragungsmittel (87-90), die so beschaffen sind, daß sie diese Drehbewegung in umgekehrter Richtung auf die mobile Walze (74) übertragen.

19. Vorrichtung nach Anspruch 18, dadurch gekennzeichnet, daß die Übertragungsorgane folgende Teile umfassen:
- eine im Verhältnis zu der Drehspindel der Hebel (77) koaxial angeordnete Zweirillenrolle (87),
- zwei Rollen (88), von denen jede mit der Drehwelle (75, 76) einer der Walzen (73, 74) fest verbunden ist,
- zwei Riemen (89, 90), von denen jeder die Rolle (88) einer der Walzen (73, 74) mit der Zweirillenrolle (87) verbindet und die so angeordnet sind, daß sie die Drehrichtung der mobilen Walze (74) im Verhältnis zu der feststehenden Walze (73) umkehren.

20. Vorrichtung nach Anspruch 5 und einem der Ansprüche 16 bis 19, dadurch gekennzeichnet, daß die Aufnahmeeinheit (62), die Einzugsmittel (73-82, 86-90) für die Hülle (19) und der Lagerbereich jeweils untereinander angeordnet sind.

21. Vorrichtung nach Anspruch 20, dadurch gekennzeichnet, daß sie ein mit einer oberen Öffnung (58) versehenes Außengehäuse (55) umfaßt, wobei das besagte Außengehäuse in der Lage ist, die Aufnahmeeinheit (62) und die Einzugsmittel (73-82, 86-90) aufzunehmen und in ihrem unteren Teil einen zugänglichen Lagerbereich abzugrenzen, wobei die besagte Aufnahmeeinheit (62) auf abmontierbare Weise in dem besagten Außengehäuse angeordnet ist.

22. Vorrichtung nach den Ansprüchen 3 und 21 gemeinsam, dadurch gekennzeichnet:
- daß die undurchlässige Hülle (25) rings um die Abrolleinheit (71) gelagert ist, so daß sie entlang der unteren Verlängerung der besagten Abrolleinheit rings um die erste aus dieser austretende Hülle (19) abgewickelt wird,
- daß die Abrolleinheit (71) einen oberen Kragen (72) aufweist, der so angeordnet ist, daß die besagte Abrolleinheit im Inneren des Außengehäuses (55) aufgehängt werden kann, und zwar ist das besagte Außengehäuse zu diesem Zweck mit Elementen (91) zur Abstützung des besagten Kragens versehen,
- daß die Aufnahmeeinheit (62) so angeordnet ist, daß sie sich auf dem Kragen (72) der Abrolleinheit (71) absetzt.

23. Vorrichtung nach Anspruch 22, dadurch gekennzeichnet, daß die Abrolleinheit (71) einen rechteckigen rohrförmigen Querschnitt aufweist und zwei Seitenflächen (71a) umfaßt, die der Länge nach konkav gestaltet sind.

24. Vorrichtung nach einem der Ansprüche 20 bis 23, dadurch gekennzeichnet:
- daß die Aufnahmeeinheit (62) einen ovoiden rohrförmigen Querschnitt aufweist und einen oberen Bereich (62a) mit sich in Richtung der Einführöffnung (63) aufweitendem zunehmendem Querschnitt sowie einen unteren Bereich (62b) mit konstantem Querschnitt umfaßt,
- daß sich die Spindeln (66, 67) in Längsrichtung der Aufnahmeeinheit (62) erstrecken, wobei die stromaufwärts befindliche Spindel (66) in dem oberen Bereich (62a) der besagten Aufnahmeeinheit und die stromabwärts befindliche Spindel (67) in dem unteren Bereich (62b) angeordnet und mit der stromaufwärts befindlichen Spindel (66) so verbunden ist, daß sie im Verhältnis zu dieser verschwenkbar ist.

25. Vorrichtung nach Anspruch 24, dadurch gekennzeichnet, daß die stromaufwärts befindliche Spindel (66) von einem Rohr (68) umgeben ist, während die untere Spindel (67) aus dem Querstück eines U besteht und die Enden der Schenkel (69) des U mit dem besagten Rohr fest verbunden sind.

26. Vorrichtung nach einem der vorstehenden Ansprüche, dadurch gekennzeichnet, daß sie eine Pumpe (51) umfaßt, die so beschaffen ist, daß sie durch die Betätigungsmittel (33) gesteuert werden kann, und die mit einem Ansaugeingang versehen ist, der mit einem Reservoir (52) als Behälter eines antiseptischen Produkts in Verbindung steht, sowie einen Förderausgang, der mit einem in der Aufnahmeeinheit (9) angeordneten Injektor (53) in Verbindung steht.

27. Eine als Abort dienende Vorrichtung nach den Ansprüchen 1, 3 und 4 gemeinsam, dadurch gekennzeichnet:
- daß die Aufnahmeeinheit (9) stromabwärts von dem Krümmer so gestaltet ist, daß sie einen Flüssigkeitserfassungsbehälter (43) bildet, wobei der besagte Behälter mit einem Ausgang zum Entleeren der besagten Flüssigkeiten versehen ist,
- daß die erste Hülle (19) eine Struktur aufweist, die so beschaffen ist, daß sie die Flüssigkeiten durch die besagte Hülle hindurch in den Erfassungsbehälter (43) abrinnen läßt,
- daß Aufbereitungsmittel (45-47) so beschaffen sind, daß sie die Aufbereitung der erfaßten Flüssigkeiten gewährleisten, um deren Beseitigung und/oder deren Verwertung zu ermöglichen.

28. Vorrichtung nach Anspruch 27, dadurch gekennzeichnet, daß die Aufbereitungsmittel Flüssigkeitszentrifugiermittel (45), Mittel (46) zum Unterdrucksetzen der nach dem Zentrifugieren erfaßten Flüssigkeiten sowie Filtrationsmittel (47) umfassen, denen die Flüssigkeiten unter Druck zugeleitet werden.

29. Vorrichtung nach Anspruch 27 oder Anspruch 28, dadurch gekennzeichnet, daß sie in einem Saugmittel (48) sowie Mittel (49, 50) zum Filtrieren der durch die besagten Saugmittel geförderten Luft umfassenden Innenraum angeordnet ist, wobei die besagten Saugmittel in der Lage sind, im Inneren des besagten Innenraums einen Unterdruck zu erzeugen.
